# EUROPEAN PATENT APPLICATION

(11) **EP 4 212 621 A1**
(43) Date of publication of application: **19.07.2023**
(21) Application number: 22151474.8
(22) Date of filing: 13.01.2022
(51) Int. Cl.: C12N 9/10, A61K 31/702, A61K 31/715, C12N 9/42, C07H 3/06, C08B 37/00, C08L 5/00

(54) **GLYCOSIDE HYDROLASE AND/OR TRANSGLYCOSYLASE FOR SYNTHESIS OF XYLOSE OLIGOSACCHARIDES**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a polypeptide with at least 85% sequence identity to SEQ ID NO: 1, as an endo-β-1,4-xylanase (EC 3.2.1.8) having transglycosylation activity. Preferably, said polypeptide is obtained from *Rhamnusium bicolor.*

In another aspect, the present invention refers to a polynucleotide encoding said polypeptide, a preparation method of said polypeptide, use of said polypeptide for producing xylose oligosaccharides by transglycosylation reaction, and a producing method of xylose oligosaccharides by using said polypeptide.

Furthermore, the present invention is directed to a mixture of xylose oligosaccharides obtainable by the producing method by using said polypeptide, and use of the mixture of said xylose oligosaccharides in life science, in particular, as prebiotics, nutraceuticals, pharmaceutically active ingredients, or food supplements.

## Description

The present invention relates to a polypeptide with at least 85% sequence identity to SEQ ID NO: 1, as an endo-β-1,4-xylanase (EC 3.2.1.8) having transglycosylation activity. Preferably, said polypeptide is obtained from *Rhamnusium bicolor.*

In another aspect, the present invention refers to a polynucleotide encoding said polypeptide, a preparation method of said polypeptide, use of said polypeptide for producing xylose oligosaccharides by transglycosylation reaction, and a producing method of xylose oligosaccharides by using said polypeptide.

Furthermore, the present invention is directed to a mixture of xylose oligosaccharides obtainable by the producing method by using said polypeptide, and use of the mixture of said xylose oligosaccharides in life science, in particular, as prebiotics, nutraceuticals, pharmaceutically active ingredients, or food supplements.

### Background of the invention

Glycoside hydrolases (GH) are classified into EC 3.2.1 as enzymes catalyzing the hydrolysis of O- or S-glycosides. The Carbohydrate-Active enZYmes Database (CAZy) classifies GHs into families according to amino acid sequence similarity. A classification system for glycosyl hydrolases, based on sequence similarity, has led to the definition of more than 100 different families. Glycoside hydrolase family 5 (GH5) comprises enzymes with several known activities including endoglucanase (EC 3.2.1.4); beta-mannanase (EC 3.2.1.78); exo-1,3-glucanase (EC 3.2.1.58); endo-1,6-glucanase (EC 3.2.1.75); xylanase (EC 3.2.1.8); endoglycoceramidase (EC 3.2.1.123).

A GH5 subfamily classification has been presented that delineates members into a number of monospecific and polyspecific clades. Subfamily GH5_2 is currently the largest in family GH5. This subfamily of extracellular enzymes, many of which are multimodular, contains a large number of characterized members that display endo-β-1,4-glucanase activity.

Xylanases belong to glycoside hydrolases subfamily GH5_2, and xylanases (endo-β-1,4-xylanase, EC 3.2.1.8) are mainly responsible for the hydrolysis of β-1,4 bonds in plant xylan. Xylanase is any of a class of enzymes that degrade the linear polysaccharide xylan into xylose, thus breaking down hemicellulose, one of the major components of plant cell walls. As such, it plays a major role in micro-organisms thriving on plant sources for the degradation of plant matter into usable nutrients. Xylanases are produced by fungi, bacteria, yeast, marine algae, protozoans, snails, crustaceans, insect, seeds, etc..

Some xylanases have been found from xylophagous long-horned beetles. To access nutrients, they secrete plant-cell-wall-degrading enzymes in their gut fluid; among them are cellulases of the subfamily 2 of glycoside hydrolase family 5 (GH5_2). Recently, it was found that several beetle-derived GH5_2s use xylan as a substrate instead of cellulose, which is unusual for this family of enzymes. A GH5_2 xylanase from the beetle *Apriona japonica* (AJAGH5_2-1) processes arabinoxylan polysaccharides in a manner distinct from classical xylanase families such as GH10 and GH11. AJAGH5_2-1 is active on long oligosaccharides and cleaves at the nonreducing end of a substituted xylose residue (Y. Pauchet et al., ChemBioChem., 2019, 21(10), pp.1517-1525).

Xylooligosaccharides (XOS) are considered as functional oligosaccharides and have great prebiotic potential. XOS are the degraded products of xylan prepared via chemical, physical or enzymatic degradation. In the enzymatic degradation, xylooligosaccharides (XOS) are usually obtained from hydrolysis of xylan by hydrolysis of xylanases. They are mainly composed of xylose units linked by b-1, 4 bonds.

Arabinoxylan oligosaccharides (AXOS) are usually obtained from hydrolysis of arabinoxylan (AX). Arabinoxylan oligosaccharides (AXOS) are useful for added-value nutraceutical products like xylitol, prebiotics, and food supplements. Deconstruction of AX by GHs leads to useful sugars for the production biofuel, especially of bioethanol.

It is the objective of the present invention to provide a polypeptide as xylanase having transglycosylation activity and a preparation method of said polypeptide. Another objective of the present invention is to provide an alternative producing method of xylose oligosaccharides by transglycosylation reaction catalyzed by said polypeptide.

A further objective of the present invention is to provide a novel mixture of xylose oligosaccharides obtainable by the producing method by using said polypeptide, and use of that mixture of said xylose oligosaccharides as prebiotics, nutraceuticals, pharmaceutically active ingredients, or food supplements.

The objectives of the present invention are solved by the teachings of the independent claims. Further advantageous features, aspects and details of the invention are evident from the dependent claims, the description, the figures, and the examples of the present application.

### Brief Description of the invention

The present invention relates to a polypeptide with at least 85% sequence identity to SEQ ID NO: 1.

Preferably, the polypeptide has at least 95% sequence identity to SEQ ID NO: 1 or SEQ ID NO: 2.

Also preferably, the polypeptide comprises an amino acid sequence SEQ ID NO: 1 or SEQ ID NO: 2.

More preferably, the polypeptide is an endo-β-1,4-xylanase (EC 3.2.1.8).

Still more preferably, the polypeptide is obtained from *Rhamnusium bicolor.*

Still more preferably, the polypeptide has transglycosylation activity.

In some embodiments, the present invention is directed to a polynucleotide encoding the polypeptide of the present invention with at least 85% sequence identity to SEQ ID NO: 3.

Preferably, the polynucleotide has at least 95% sequence identity to SEQ ID NO: 3, SEQ ID NO: 4 or SEQ ID NO:5.

In some embodiments, the present invention is directed to a nucleic acid construct or expression vector comprising the polynucleotide as described herein.

In some embodiments, the present invention is directed to a recombinant host cell comprising said polynucleotide, or said nucleic acid construct or expression vector as described herein.

In some embodiments, the present invention is directed to a method for obtaining said polypeptide comprising:
A) providing a polynucleotide as described herein,
B) cloning said polynucleotide into a nucleic acid construct or expression vector,
C) transfecting said vector obtained by step B) into recombinant host cells;
D) culturing said recombinant host cells obtained by step C);
E) isolating the polypeptide of invention from said cultured recombinant host cells.

Preferably, in the inventive method the recombinant host cells are Sf9 cells.

In some embodiments, the present invention is directed to use of said polypeptide for producing xylose oligosaccharides by transglycosylation reaction.

Preferably, said xylose oligosaccharides are xylooligosaccharides (XOS), arabinoxylan oligosaccharides (AXOS), or fragments thereof.

In some embodiments, the present invention is directed to a method for producing xylose oligosaccharides comprising:
performing transglycosylation reaction of a glycosyl donor with a glycosyl acceptor by using the polypeptide of any one of the present invention,
wherein the glycosyl donor is selected from the group consisting of: xylose, xylobiose, xylotriose, xylotetraose, xylopentaose, xylohexaose and xyloheptaose; and
the glycosyl acceptor is a substrate containing at least 2 xylose units.

Preferably, in said method as described herein, the substrate containing at least 2 xylose units has the formula (II): 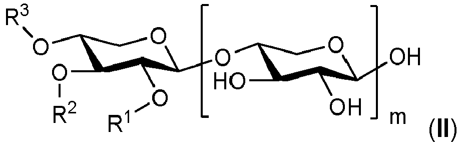 wherein,
**m** is an integer selected from 1 to 6;
**R¹** represents-H, or 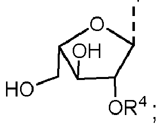
**R²** represents -H, or 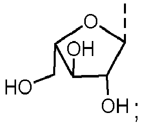
**R³** represents -H, or 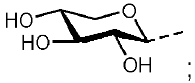
**R⁴** represents -H, or

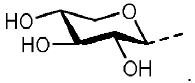

Preferably, in said method as described herein, each xylose oligosaccharide has the formula (I): 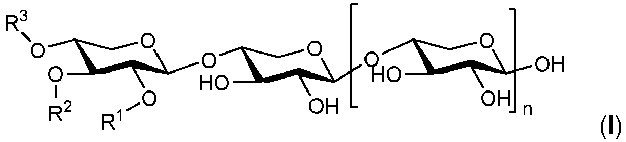 wherein
n is an integer selected from 1 to 18;
**R¹** represents-H, or 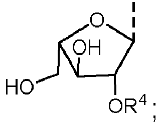
**R²** represents -H, or 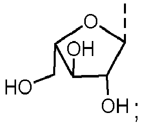
**R³** represents -H, or 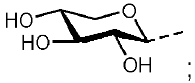
**R⁴** represents -H, or

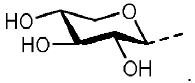

In some embodiments, the present invention relates to a mixture of xylose oligosaccharides obtainable by said method as described herein.

Preferably, the xylose oligosaccharides are xylooligosaccharides (XOS), arabinoxylan oligosaccharides (AXOS), or fragments thereof.

More preferably, each xylose oligosaccharide has the formula (I): 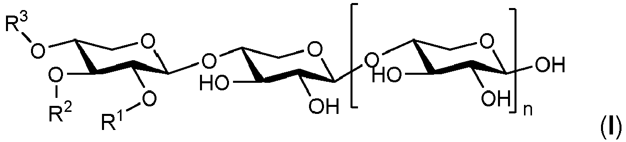 wherein
n is an integer selected from 1 to 18;
**R¹** represents-H, or 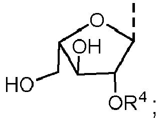
**R²** represents -H, or 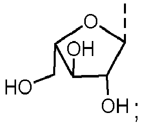
**R³** represents -H, or 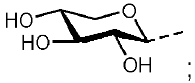
**R⁴** represents -H, or

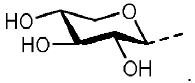

Said mixture of xylose oligosaccharides is used in life science.

Preferably, said mixture of xylose oligosaccharides is useful as prebiotics, nutraceuticals, pharmaceutically active ingredients, or food supplements.

Preferably, said mixture of xylose oligosaccharides is useful in prophylaxis and/or treatment of bacterial infectious disease, cancer, immune disease, type 2 diabetes, or cardiovascular disease.

### Description of the invention

The present invention relates to a polypeptide with at least 85% sequence identity to SEQ ID NO: 1; preferably at least 90%, more preferably at least 93%, still more preferably at least 95%, still more preferably at least 96%, still more preferably at least 97%, still more preferably at least 98% sequence identity to SEQ ID NO: 1.

The term "sequence identity" as used herein is the number of characters which match exactly between two different sequences, whereby gaps are not counted and the measurement is relational to the shorter sequence of the two aligned sequences.

Preferably, said polypeptide comprises at least one amino acid selected from Arg85, Asn159, Glu160, Tyr199, Phe199, His220, Tyr222, Thr248, Trp283.

Especially said polypeptide comprises at least Thr248. The polypeptide of the present invention differs from the other GH5 family in that said polypeptide comprises Thr248 and other known GH5 family xylanases comprise Ser248 instead of Thr248.

More preferably, said polypeptide comprises amino acids Arg85, Asn159, Glu160, His220, Tyr222, Thr248, and Trp283, and optionally Tyr199, or Phe199.

In some aspects, said polypeptide further comprises at least one amino acid selected from the group consisting of: Ile7, Ala10, Asn38, Ile40, Thr43, Asn92, Glu104, Ser127, Ser129, Glu135, Ser136, Arg149, Gln164, Gln175, Tyr201, Ile205, Asn206, Arg213, Gly226, Gly227, Asn228, Asp229, Gln230, Asp236, Ser240, Ala241, Ser253, Met254, Ala265, Val268, Gly277, Ile278, and Asn308; preferred at least two amino acids, or at least three amino acids, preferred, at least four amino acids, preferably at least five amino acids, more preferred,.

Preferably, the polypeptide has at least 95% sequence identity to SEQ ID NO: 1 or SEQ ID NO: 2, more preferably at least 96%, still more preferably at least 97%, still more preferably at least 98% sequence identity to SEQ ID NO: 1 or SEQ ID NO: 2. Also preferably, the polypeptide comprises an amino acid sequence SEQ ID NO: 1 or SEQ ID NO: 2.

In some embodiments, the polypeptide of the present invention is an endo-β-1,4-xylanase (EC 3.2.1.8). Preferably, said polypeptide is obtained from *Rhamnusium bicolor.*

Surprisingly, it was found that the polypeptide of the present invention has not only hydrolysis activity, but also transglycosylation activity. Especially, the polypeptide of the present invention favours transglycosylation over hydrolysis. Thus, in some embodiments, the polypeptide has transglycosylation activity.

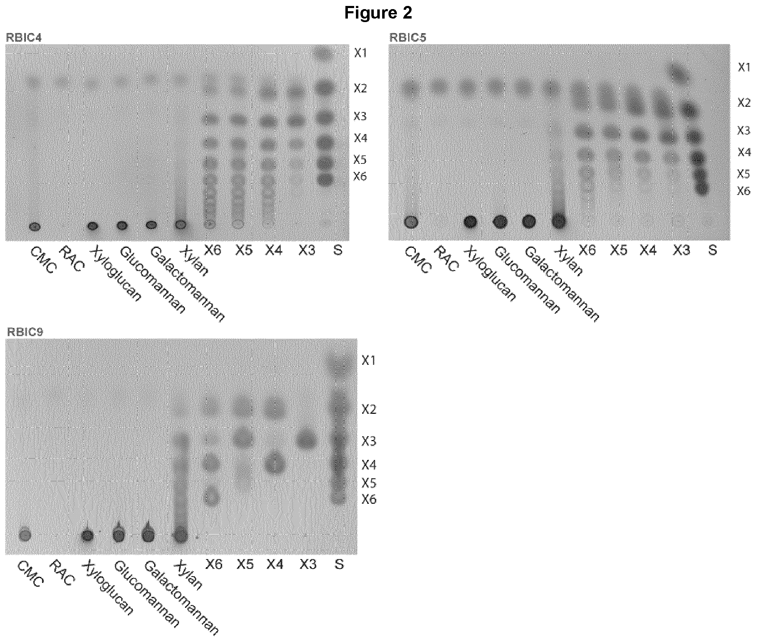

As summarized the results presented in **Fig. 2**, Rbic9 (SEQ ID NO: 6) as GH5 breaks down xylan poly- and oligosaccharides, but none of the other polysaccharides tested and is thus an endo-β-1,4-xylanase. Rbic9 breaks down xylotetraose (X4) and bigger oligosaccharides, but is unable to break down xylotriose and smaller oligosaccharides.

However, Rbic4 (SEQ ID NO: 1) as a polypeptide of the present invention, does not break down any of the polysaccharides tested, including xylan. However, when xylotetraose (X4), xylopentaose (X5) and xylohexaose (X6) were used as substrates, Rbic4 generates a range of xylooligosaccharides - *several of them longer than the original substrate* - from xylobiose to at least xylodecaose (forming a "ladder-like" range of xylooligosaccharides), *indicating that Rbic4 possesses reduced hydrolytic, but strong transglycosylation activity on these xylooligosaccharides.* In addition, Rbic4 shows some level of activity using xylotriose as substrate with the formation of xylooligosaccharides ranging from xylobiose to xyloheptaose or xylooctaose.

As another polypeptide of the present invention, Rbic5 (SEQ ID NO: 2) shows an "intermediate" pattern between Rbic4 (SEQ ID NO: 1) and Rbic9 (SEQ ID NO: 6). Although breakdown products are clearly visible when a xylan polysaccharide is used as substrate, some transglycosylation products (oligosaccharides larger than the substrate used) are clearly visible when xylotriose, xylotetraose, xylopentaose and xylohexaose are used as substrates.

### Influence of the ratio enzyme/substrate on the enzymatic activity of Rbic4, Rbic5 and Rbic9

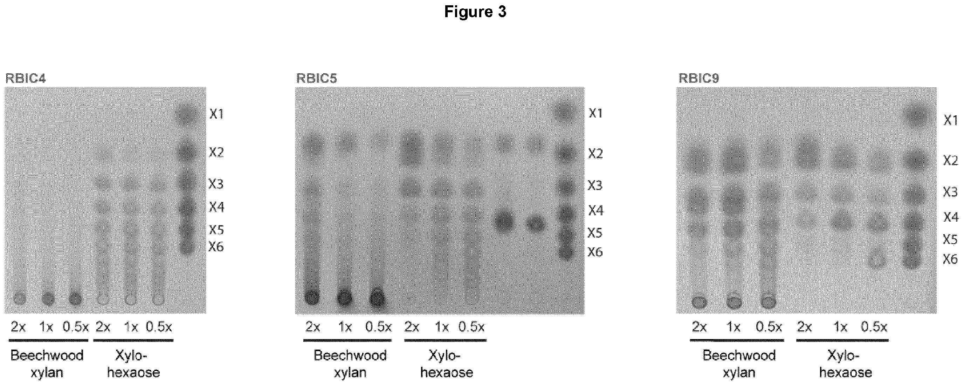

To test whether the ratio enzyme/substrate can influence the enzymatic activity of Rbic4, Rbic5 and Rbic9, we performed activity assays similar to those presented in **Fig. 2**, where we use three different amounts of enzyme with a constant amount of substrate. We tested a xylan polysaccharide as well as xylohexaose (**Fig. 3**). The results are summarized as follows and presented in **Fig. 3**:
- An increasing amount of Rbic4 does not have obvious effect on its transglycosylation activity with xylohexaose as substrate and does not improve its activity on xylan polysaccharide.
- An increasing amount of Rbic9 increases its efficiency in hydrolysing xylan poly- and oligosaccharides.
- An increasing amount of Rbic5 increases its hydrolysing activity on xylan polysaccharide. Interestingly, Rbic5 seems to favour transglycosylation at low enzyme amount (0.5x and 1x), but hydrolysis seems to be favoured at the highest amount of enzyme tested (2x) with xylohexaose as substrate.

### Influence of the incubation time on the activity of Rbic4

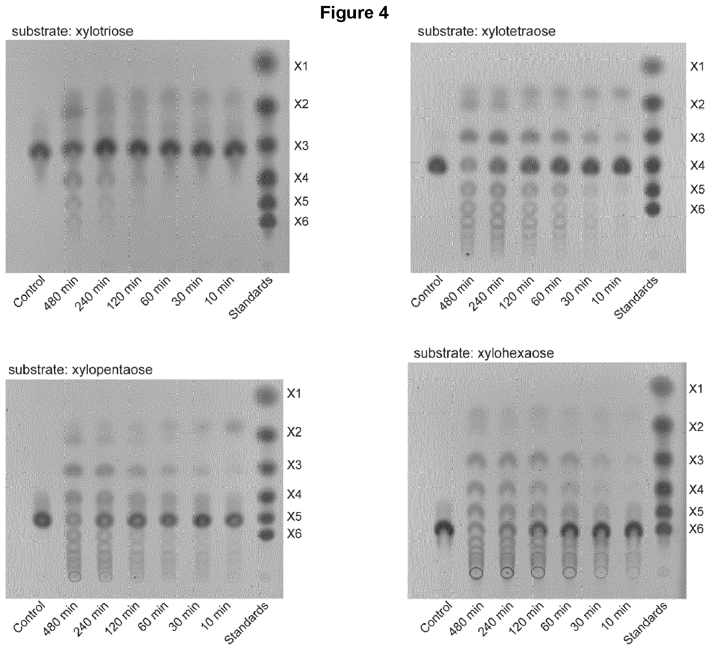

Furthermore, the effects of incubation time on the enzymatic activity of Rbic4 have been tested on four xylan oligosaccharides **(****Fig. 4**). The production of long xylan oligosaccharides larger than the substrate used occurs even at short incubation times. It was also confirmed that the transglycosidase activity of Rbic4 is high with xylotetraose, xylopentaose and xylohexaose, but not so effective on xylotriose **(****Fig. 4**).

Compared to Rbic9, which is a typical xylanase, a polypeptide of the present invention, Rbic4 is a naturally occurring enzyme favouring transglycosylation over hydrolysis, and can be used for the synthesis of a range of xylooligosaccharides including very long ones. From one enzymatic reaction using a single xylooligosaccharide as substrate, for example xylohexaose, a range of xylooligosaccharides from xylobiose to very long ones, at least xylodecaose, are produced. These xylooligosaccharides could eventually be purified individually using for example preparative chromatography. Rbic5 also possesses the ability to synthesize long xylooligosaccharides, but seems more sensitive than Rbic4 to the reaction conditions, especially the ratio enzyme/substrate.

Therefore, preferably, the polypeptide of the present invention favours transglycosylation over hydrolysis and it has a ratio of transglycosylation to hydrolysis in a range of 1.5 to 50, preferably 2.0 to 50, preferably 3.0 to 20, more preferably 4.0 to 15, most preferably 5.0 to 10.0.

A known GH5_2 xylanases, Ajap1 (SEQ ID NO:8) from the beetle *Apriona japonica* (AJAGH5_2-1) and Agla1 (SEQ ID NO:10) from Anoplophora glabripennis as references are merely active on long oligosaccharides and cleaves at the nonreducing end of a substituted xylose residue.

Thus, the invention relates to a polypeptide with at least 85% sequence identity to SEQ ID NO: 1 and having transglycosylation activity; preferably a polypeptide with at least 90% sequence identity to SEQ ID NO: 1, and having transglycosylation activity. Preferably, said polypeptide comprises at least one amino acid selected from Arg85, Asn159, Glu160, Tyr199, Phe199, His220, Tyr222, Thr248, Trp283.

Preferably, said polypeptide comprises at least one amino acid selected from Arg85, Especially said polypeptide comprises at least Thr248. The polypeptide of the present invention differs from the other GH5 family in that said polypeptide comprises Thr248 and other known GH5 family xylanases comprise Ser248 instead of Thr248.

More preferably, said polypeptide comprises amino acids Arg85, Asn159, Glu160, His220, Tyr222, Thr248, and Trp283, and optionally Tyr199, or Phe199.

In some aspects, said polypeptide further comprises at least one amino acid selected from the group consisting of: Ile7, Ala10, Asn38, Ile40, Thr43, Asn92, Glu104, Ser127, Ser129, Glu135, Ser136, Arg149, Gln164, Gln175, Tyr201, Ile205, Asn206, Arg213, Gly226, Gly227, Asn228, Asp229, Gln230, Asp236, Ser240, Ala241, Ser253, Met254, Ala265, Val268, Gly277, Ile278, and Asn308; preferred at least two amino acids, or at least three amino acids, preferred, at least four amino acids, preferably at least five amino acids, more preferred,.

In particular, said polypeptide having transglycosylation activity is selected from the group consisting of:
(a) a polypeptide having at least 95% sequence identity to SEQ ID NO: 1; and
(b) a polypeptide having at least 95% sequence identity to SEQ ID NO: 2; preferably,

(a) a polypeptide having the sequence identity to SEQ ID NO: 1; and
(b) a polypeptide having the sequence identity to SEQ ID NO: 2;
   more preferably, the polypeptide of the present invention is an endo-β-1,4-xylanase (EC 3.2.1.8). Preferably, said polypeptide is obtained from *Rhamnusium bicolor.*

In some aspects, said polypeptide as described above has a ratio of transglycosylation to hydrolysis in a range of 1.5 to 50, preferably 2.0 to 50, preferably 3.0 to 20, more preferably 4.0 to 15, most preferably 5.0 to 10.0.

Preferably, a polypeptide comprises the polypeptide of (a), (b) and further an N-terminal and/or C-terminal His-tag and/or HQ-tag.

In some embodiments, the present invention is directed to a polynucleotide encoding the polypeptide of the invention with at least 85% sequence identity to SEQ ID NO: 3. Preferably, the polynucleotide has at least 95% sequence identity to SEQ ID NO: 3, SEQ ID NO: 4 or SEQ ID NO:5.

In some embodiments, the present invention is directed to a nucleic acid construct or expression vector comprising the polynucleotide of the invention.

In some embodiments, the present invention is directed to a recombinant host cell comprising said polynucleotide, or said nucleic acid construct or expression vector as described herein.

Expression: The term "expression" includes any step involved in the production of a polypeptide including, but not limited to, transcription, post-transcriptional modification, translation, post-translational modification, and secretion.

Expression vector: The term "expression vector" means a linear or circular DNA molecule that comprises a polynucleotide encoding a polypeptide and is operably linked to control sequences that provide for its expression.

In some embodiments, the present invention is directed to a method for obtaining said polypeptide comprising:
A) providing a polynucleotide of the invention,
B) cloning said polynucleotide into a nucleic acid construct or expression vector,
C) transfecting said vector obtained by step B) into recombinant host cells;
D) culturing said recombinant host cells obtained by step C);
E) isolating the polypeptide of invention from said cultured recombinant host cells.

Preferably, said polynucleotide encoding the polypeptide of the invention has at least 85% sequence identity to SEQ ID NO: 3; more preferably, at least 90% sequence identity to SEQ ID NO: 3. Still more preferably, said polynucleotide encoding the polypeptide of the invention has at least 95% sequence identity to SEQ ID NO: 3, SEQ ID NO: 4 or SEQ ID NO: 5.

Most preferably, said polynucleotide encoding the polypeptide of the invention has a nucleotide sequence of SEQ ID NO: 3, a nucleotide sequence of SEQ ID NO: 4 or a nucleotide sequence of SEQ ID NO: 5.

Host cell: The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

Preferably, in the inventive method, the recombinant host cells are Sf9 cells.

In some embodiments, the present invention is directed to use of said polypeptide for producing xylose oligosaccharides by transglycosylation reaction.

Preferably, said xylose oligosaccharides are xylooligosaccharides (XOS), arabinoxylan oligosaccharides (AXOS), or fragments thereof.

Isolated: the term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance).

It is known in the art that a host cell may produce a mixture of two of more different mature polypeptides (i.e., with a different C-terminal and/or N-terminal amino acid) expressed by the same polynucleotide. It is also known in the art that different host cells process polypeptides differently, and thus, one host cell expressing a polynucleotide may produce a different mature polypeptide (e.g., having a different C-terminal and/or N-terminal amino acid) as compared to another host cell expressing the same polynucleotide.

Mature polypeptide coding sequence: The term "mature polypeptide coding sequence" means a polynucleotide that encodes a mature polypeptide having transglycosylation activity. In one aspect, the mature polypeptide coding sequence is nucleotides of SEQ ID NO: 3 to 5.

Mature polypeptide: The term "mature polypeptide" means a polypeptide in its final form following translation and any post-translational modifications, such as N-terminal processing, C-terminal truncation, glycosylation, phosphorylation, etc. In one aspect, the mature polypeptide comprises at least 85% amino acids of SEQ ID NO: 1; preferably, at least 90% amino acids of SEQ ID NO: 1, more preferably, at least 90% amino acids of SEQ ID NO: 1, or at least 90% amino acids of SEQ ID NO: 2; more preferably at least 95% amino acids of SEQ ID NO: 1, or at least 95% amino acids of SEQ ID NO: 2; still more preferably at least 98% amino acids of SEQ ID NO: 1, or at least 98% amino acids of SEQ ID NO: 2; most preferably, the mature polypeptide comprises amino acids 1 to 323 of SEQ ID NO: 1, or 1 to 323 of SEQ ID NO: 2.

In some embodiments, the present invention is directed to a method for producing xylose oligosaccharides comprising:
performing transglycosylation reaction of a glycosyl donor with a glycosyl acceptor by using the polypeptide of the invention,
wherein the glycosyl donor is selected from the group consisting of: xylose, xylobiose, xylotriose, xylotetraose, xylopentaose, xylohexaose and xyloheptaose; and
the glycosyl acceptor is a substrate containing at least 2 xylose units.

The glycosyl donors, xylose (**1**), xylobiose (**2**), xylotriose (**3**), xylotetraose (**4**), xylopentaose (**5**), xylohexaose (**6**), and xyloheptaose (**7**) have each the following structure:

### Glycosyl donor for synthesis of Xylooligosaccharides (XOS):

| | |
|---|---|
| **1** : Xylose (X) | **2** : Xylobiose (X₂) |
| 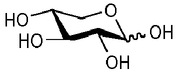 | 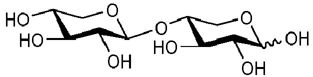 |
| **3** : Xylotriose (X₃) | **4** : Xylotetraose (X₄) |
| 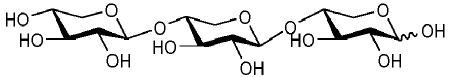 | 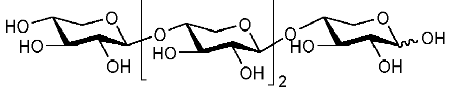 |
| **5** : Xylopentaose (X₅) | **6** : Xylohexaose (X₆) |
| 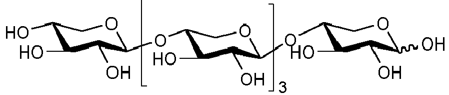 | 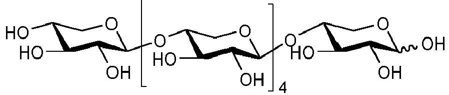 |
| **7** : Xyloheptaose (X₇) | |
| 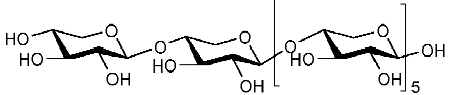 | |

Preferably, in said method as described herein, the glycosyl donor is selected from the group consisting of: xylotriose (**3**), xylotetraose (**4**), xylopentaose (**5**), xylohexaose (**6**), and xyloheptaose (**7**), more preferably, xylotriose (**3**), xylotetraose (**4**), xylopentaose (**5**), xylohexaose (**6**), and xyloheptaose (**7**); still more preferred, xylotetraose (**4**), xylopentaose (**5**), and xylohexaose (**6**).

In some embodiments, in said method as described herein, the substrate containing at least 2 xylose units has the formula (II): 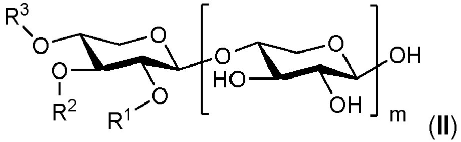 wherein,
**m** is an integer selected from 1 to 6;
**R¹** represents-H, or 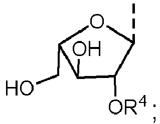
**R²** represents -H, or 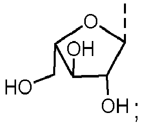
**R³** represents -H, or 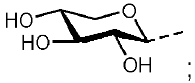
**R⁴** represents -H, or

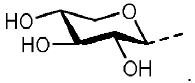

Preferably, in said method as described herein, each xylose oligosaccharide has the formula (I): 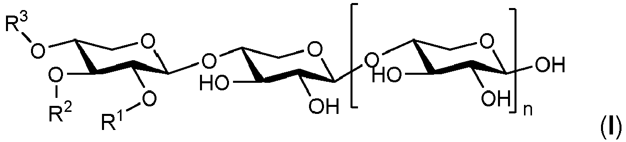 wherein
n is an integer selected from 1 to18;
**R¹** represents-H, or 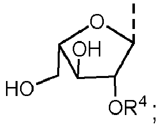
**R²** represents -H, or 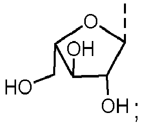
**R³** represents -H, or 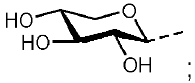
**R⁴** represents -H, or

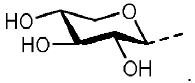

### For producing xylooligosaccharides (XOS)

As glycosyl acceptor, any one of compounds **2** to **7** (X₂ to X₇) is provided for producing the xylooligosaccharides (XOS): Preferably one of compounds **2** to **6** (X₂ to X₆) more preferably X₃ to X₆, most preferably X₄ to X₆ is provided for producing the xylooligosaccharides (XOS).

| | |
|---|---|
| **2** : Xylobiose (X₂) | **3** : Xylotriose (X₃) |
| 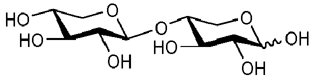 | 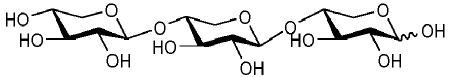 |
| **4** : Xylotetraose (X₄) | **5** : Xylopentaose (X₅) |
| 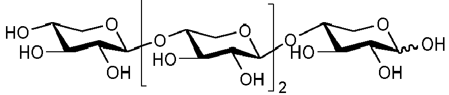 | 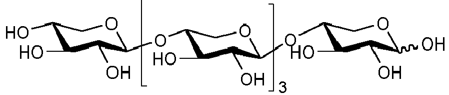 |
| **6 :** Xylohexaose (X₆) | **7 :** Xyloheptaose (X₇) |
| 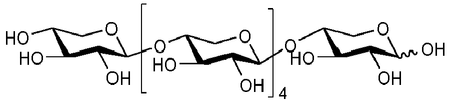 | 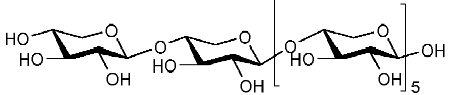 |

Thus, in one aspect, the present invention relates to a method for producing xylose oligosaccharides comprising:
performing transglycosylation reaction of a glycosyl donor with a glycosyl acceptor by using the polypeptide of the invention,
   wherein
the glycosyl donor is selected from the group consisting of: xylose, xylobiose, xylotriose, xylotetraose, xylopentaose, xylohexaose and xyloheptaose; and
the glycosyl acceptor is a substrate containing at least 2 xylose units, and
the substrate containing at least 2 xylose units has the formula (II): 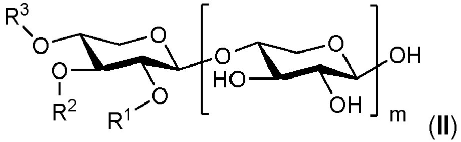 wherein,
   **m** is an integer selected from 1 to 6;
      preferably, 2 to 5, more preferably 3 to 5;
   **R¹** represents-H;
   **R²** represents -H;
   **R³** represents -H, or 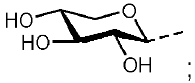
   **R⁴** represents -H.

Preferably, the glycosyl donors is selected from xylotriose (**3**), xylotetraose (**4**), xylopentaose (**5**), and xylohexaose (**6**).

Preferably, in said method as described herein, each xylose oligosaccharide has the formula (I): 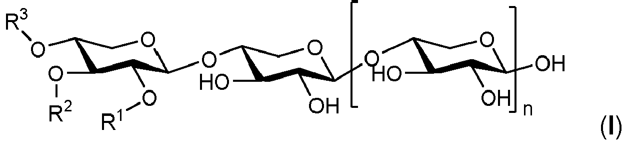 wherein
n is an integer selected from 1 to18; preferably, 1 to 16, more preferably 1 to 14; still more preferably, 1 to 12, and most preferably 1 to 10;
**R¹** represents-H;
**R²** represents -H;
**R³** represents -H, or 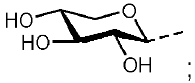
**R⁴** represents -H.

More preferably, the following xylose oligosaccharides (XOS) **3 - 20** are produced by the preparation method of the present invention as described above.

| | |
|---|---|
| **3** :Xylotriose (X₃) | **4** :Xylotretraose (X₄) |
| 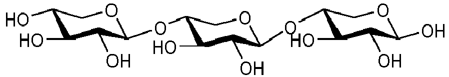 | 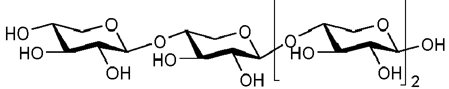 |
| **5** :Xylopentaose (X₅) | **6** :Xylohexaose (X₆) |
| 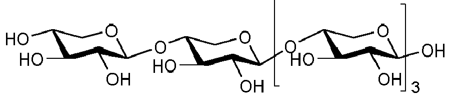 | 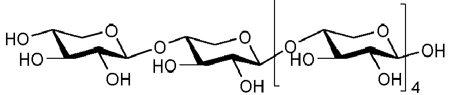 |
| **7** : Xyloheptaose (X₇) | **8** : Xylooctaose (X₈) |
| 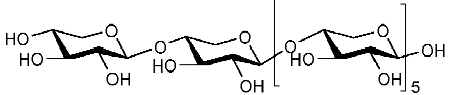 | 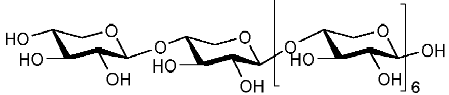 |
| **9** : Xylononaose (X₉) | **10** : Xylodecaose (X₁₀) |
| 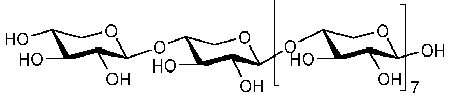 | 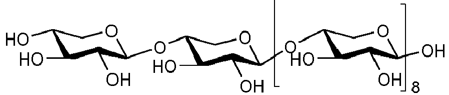 |
| **11** : Xyloundecaose (X₁₁) | **12** : Xylododecaose (X₁₂) |
| 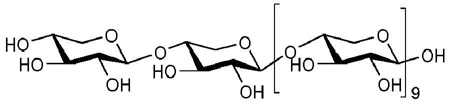 | 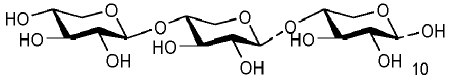 |
| **13** : Xylotridecaose (X₁₃) | **14** : Xylotetradecaose (X₁₄₎ |
| 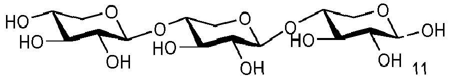 | 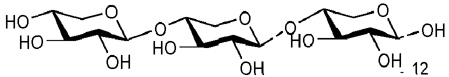 |
| **15** : Xylopentadecaose (X₁₅) | **16** : Xylohexadecaose (X₁₆) |
| 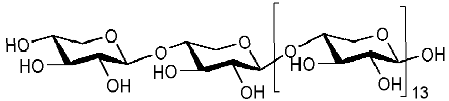 | 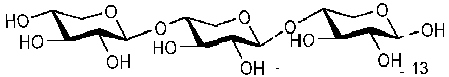 |
| **17** : Xyloheptadecaose (X₁₇) | **18** : Xylooctadecaose (X₁₈) |
| 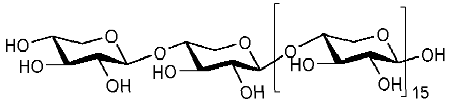 | 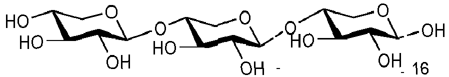 |
| **19** : Xylononadecaose (X₁₉) | **20** : Xyloicosaose (X₂₀) |
| 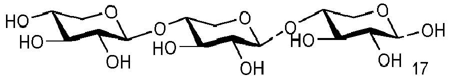 | 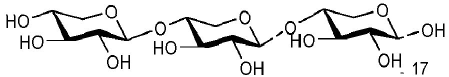 |

### For producing arabinoxylan oligosaccharides (AXOS)

For producing the arabinoxylan oligosaccharides (AXOS), as glycosyl acceptor, any one of the following compounds **21** to **46** is provided. Preferably, one of the compounds **21** to **28**, and **35** to **37**; more preferably compounds **21** to **28**, most preferably compounds **22** to **25** is provided for producing the arabinoxylan oligosaccharides (AXOS).

| | |
|---|---|
| **21:** (A²X) | **22:** (A³X) |
| 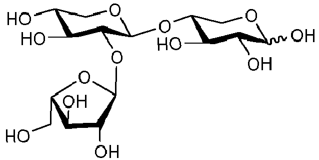 | 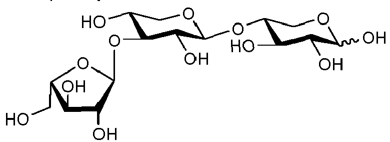 |
| **23:** (A²X₂) | **24:** (A³X₂) |
| 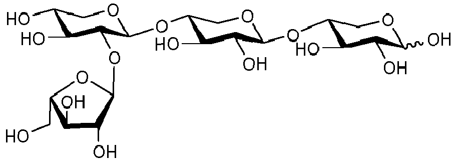 | 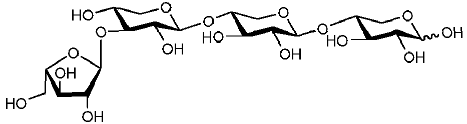 |
| **25:** (XA³X) | **26:** (XA²X) |
| 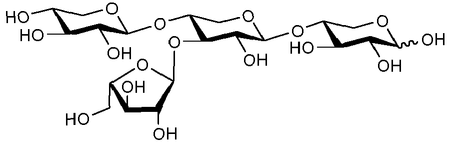 | 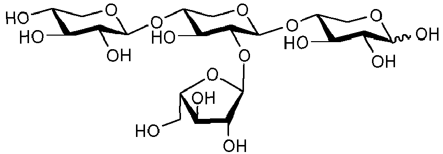 |
| **27:** (A³X₃) | **28:** (A²X₃) |
| 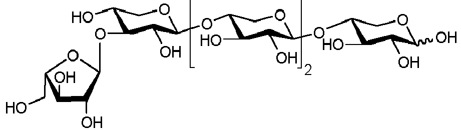 | 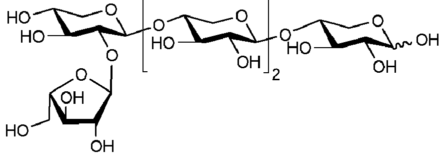 |
| **29:** (A³X₄) | **30:** (A²X₄) |
| 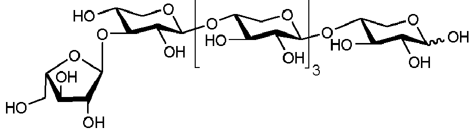 | 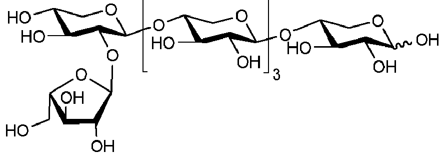 |
| **31:** (A³X₅) | **32:** (A²X₅) |
| 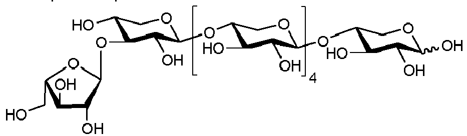 | 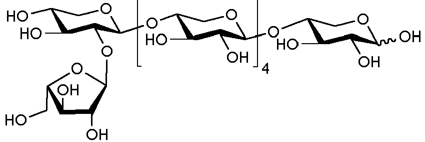 |
| **33:** (A³X₆) | **34:** (A²X₆) |
| 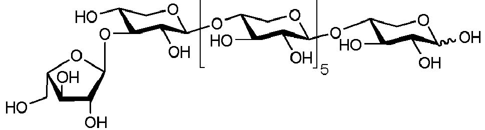 | 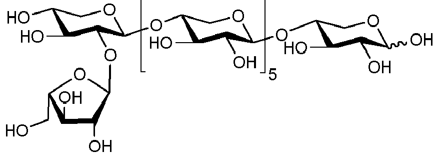 |
| **35:** (A²⁺³X) | **36:** (A²⁺³X₂) |
| 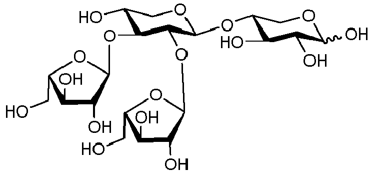 | 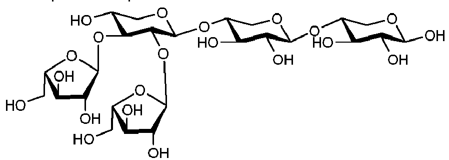 |
| **37:** (A²⁺³X₃) | 38: (A²⁺³X₄) |
| 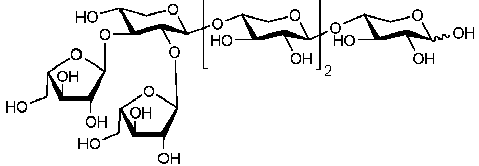 | 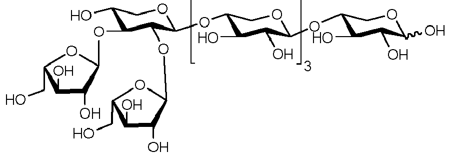 |
| **39:** (A²⁺³X₅) | **40:** (A²⁺³X₆) |
| 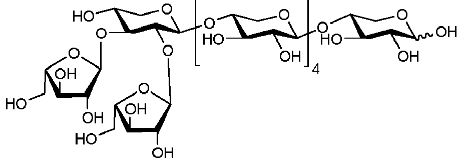 | 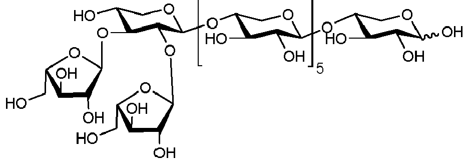 |
| **41:** (XA²⁺³X) | **42:** (XA²⁺³X₂) |
| 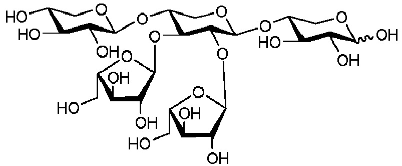 | 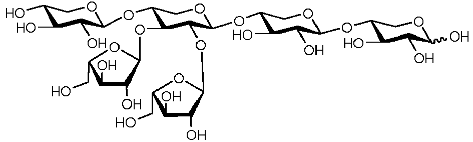 |
| **43:** (XA²⁺³X₃) | **44:** (XA²⁺³X₄) |
| 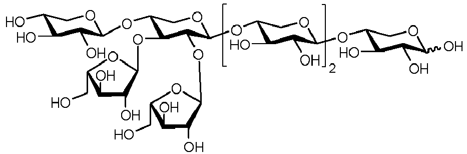 | 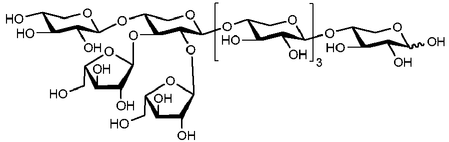 |
| **45:** (XA²⁺³X₅) | **46:** (XA²⁺³X₆) |
| 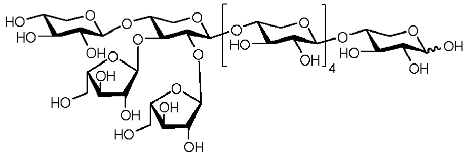 | 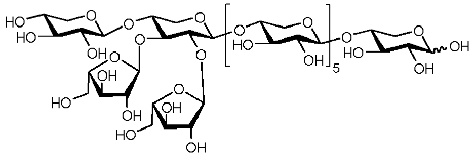 |

Preferably, the following arabinoxylan oligosaccharides (AXOS) **23** - **130** are produced by the preparation method of the present invention as described above.

| | |
|---|---|
| **23:** A²X₂ | **24:** (A³X₂) |
| 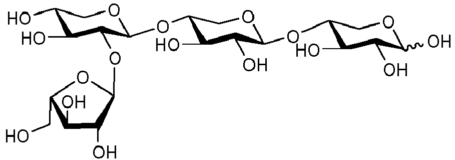 | 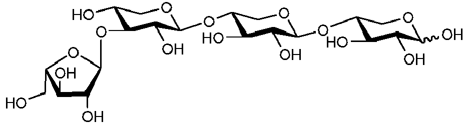 |
| **25:** (XA³X) | **26:** (XA²X) |
| 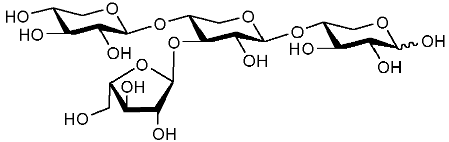 | 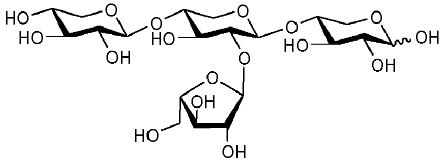 |
| **27:** (A³X₃) | **28:** (A²X₃) |
| 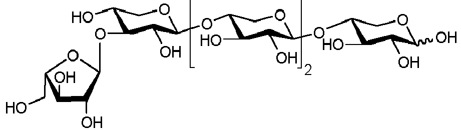 | 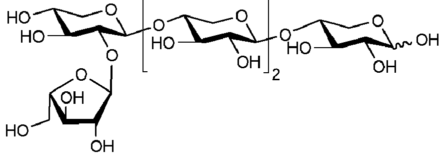 |
| **29:** (A³X₄) | **30:** (A²X₄) |
| 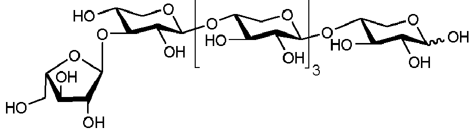 | 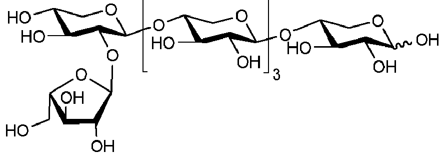 |
| **31:** (A³X₅) | **32:** (A²X₅) |
| 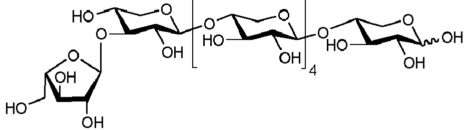 | 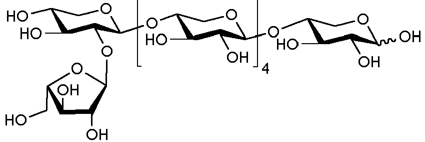 |
| **33:** (A³X₆) | **34:** (A²X₆) |
| 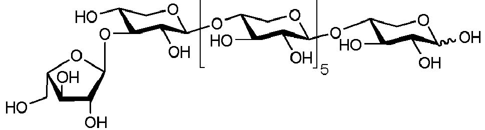 | 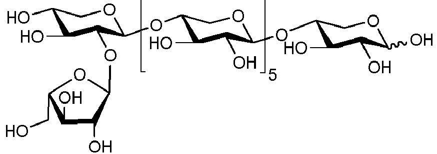 |
| **35:** (A²⁺³X) | **36:** (A²⁺³X₂) |
| 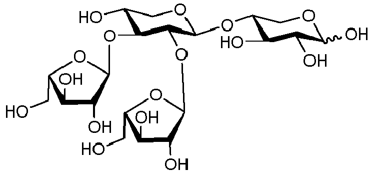 | 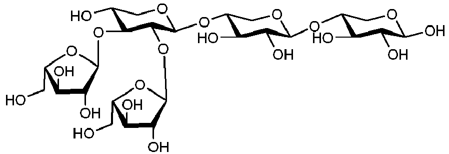 |
| **37:** (A²⁺³X₃) | **38:** (A²⁺³X₄) |
| 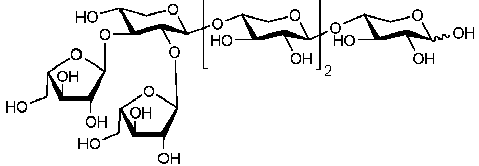 | 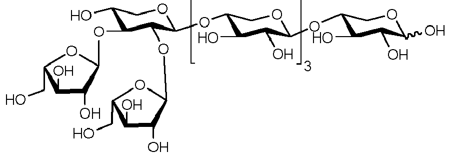 |
| 39: (A²⁺³X₅) | **40:** (A²⁺³X₆) |
| 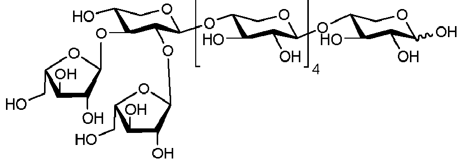 | 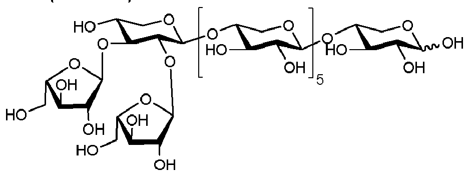 |
| **41:** (XA²⁺³X) | **42:** (XA²⁺³X₂) |
| 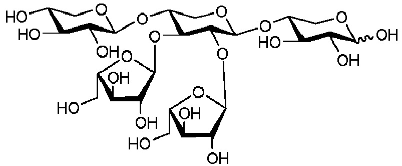 | 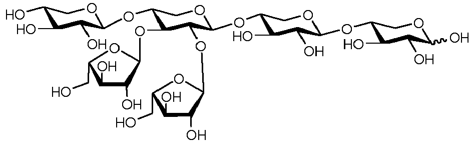 |
| **43:** (XA²⁺³X₃) | **44:** (XA²⁺³X₄) |
| 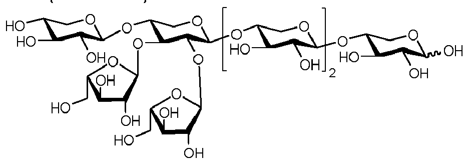 | 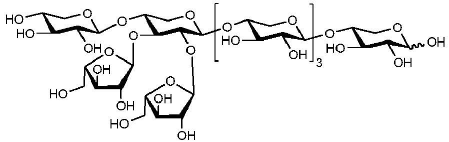 |
| **45:** (XA²⁺³X₅) | **46:** (XA²⁺³X₆) |
| 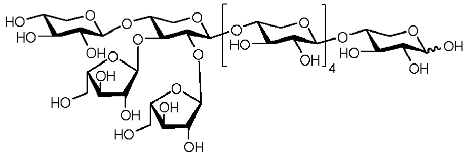 | 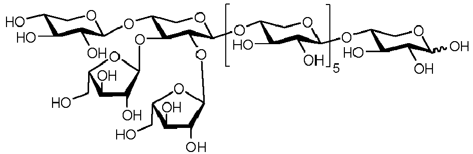 |
| **47:** (A²X₇) | **48:** (A²X₈) |
| 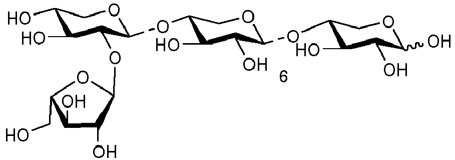 | 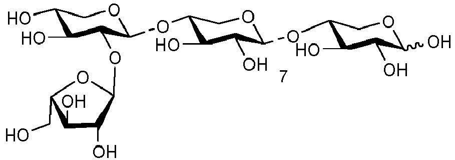 |
| **49:** (A²X₉) | **50:** (A²X₁₀) |
| 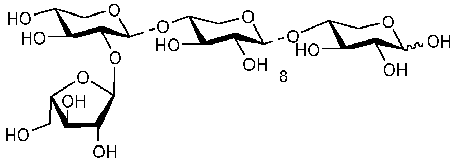 | 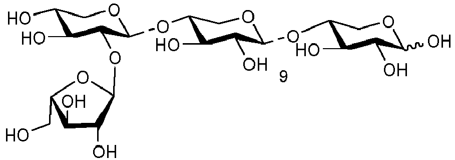 |
| **51:** (A²X₁₁) | **52:** (A²X₁₂) |
| 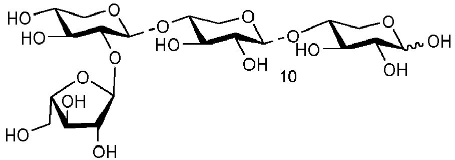 | 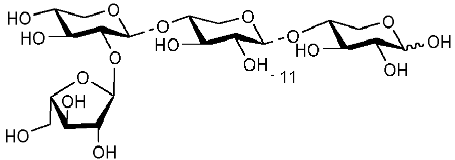 |
| 53: (A²X₁₃) | **54:** (A²X₁₄) |
| 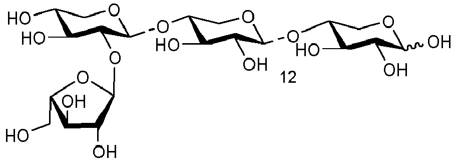 | 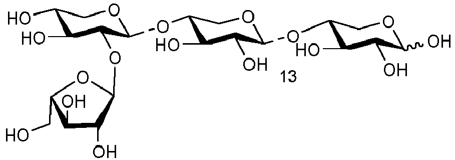 |
| **55:** (A²X₁₅) | **56:** (A²X₁₆) |
| 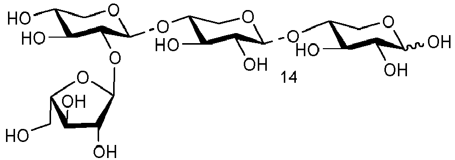 | 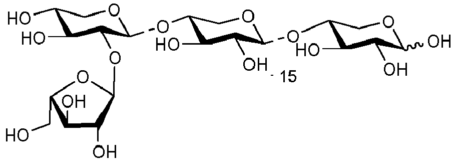 |
| **57:** (A²X₁₇) | **58:** (A²X₁₈) |
| 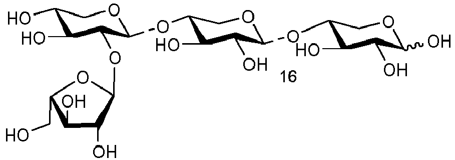 | 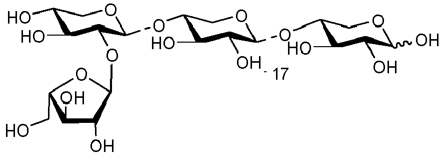 |
| **59:** (A²X₁₉) | **60:** (A²X₂₀) |
| 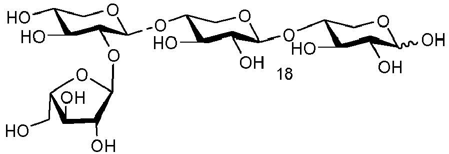 | 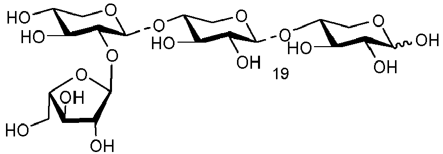 |
| **61:** (A³X₇) | **62:** (A³X₈) |
| 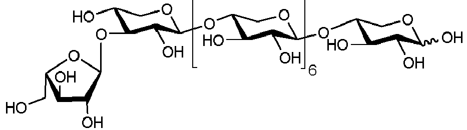 | 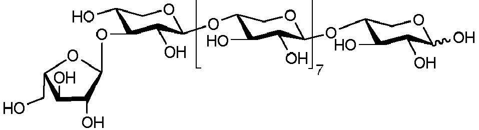 |
| **63:** (A³X₉) | **64:** (A³X₁₀) |
| 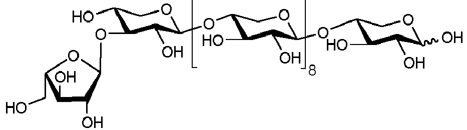 | 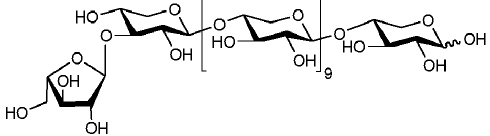 |
| **65:** (A³X₁₁) | **66:** (A³X₁₂) |
| 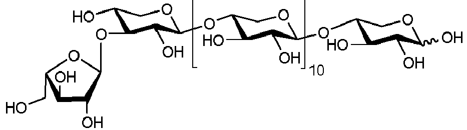 | 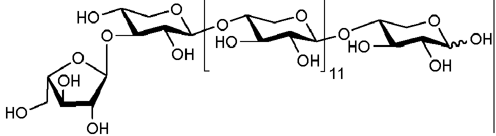 |
| **67:** (A³X₁₃) | **68:** (A³X₁₄) |
| 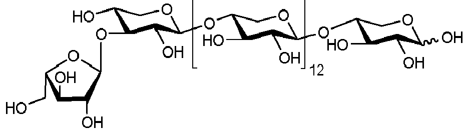 | 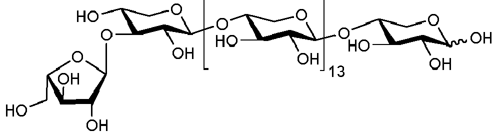 |
| **69:** (A³X₁₅) | **70:** (A³X₁₆) |
| 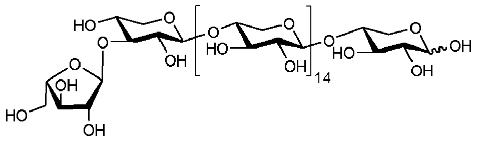 | 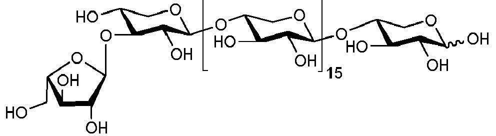 |
| **71:** (A³X₁₇) | **72:** (A³X₁₈) |
| 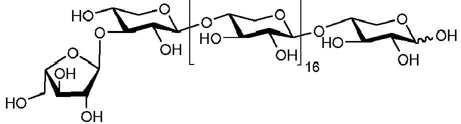 | 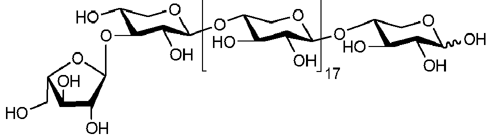 |
| **73:** (A³X₁₉) | **74:** (A³X₂₀) |
| 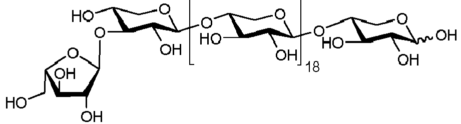 | 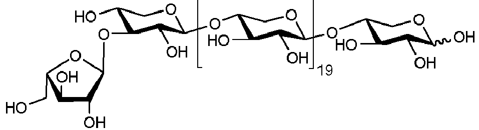 |
| **75:** (A²⁺³X₇) | **76:** (A²⁺³X₈) |
| 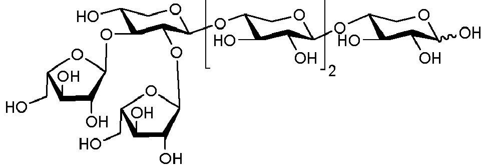 | 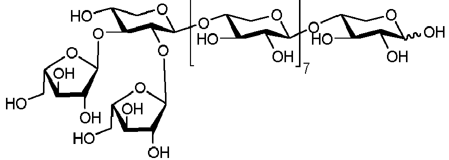 |
| **77:** (A²⁺³X₉) | **78:** (A²⁺³X₁₀) |
| 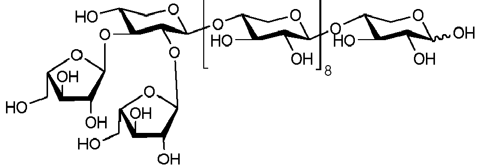 | 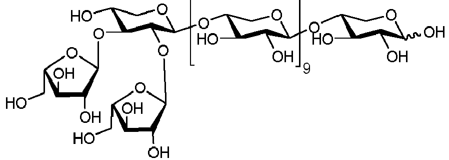 |
| **79:** (A²⁺³X₁₁) | **80:** (A²⁺³X₁₂) |
| 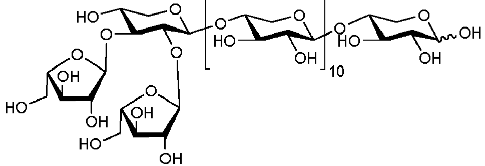 | 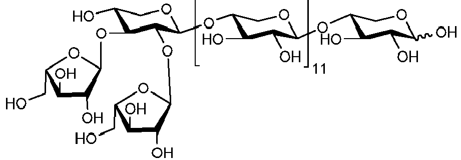 |
| **81:** (A²⁺³X₁₃) | **82:** (A²⁺³X₁₄) |
| 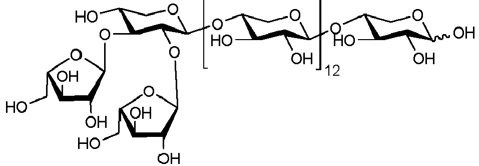 | 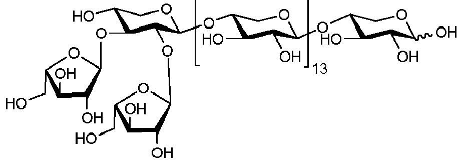 |
| **83:** (A²⁺³X₁₅) | **84:** (A²⁺³X₁₆) |
| 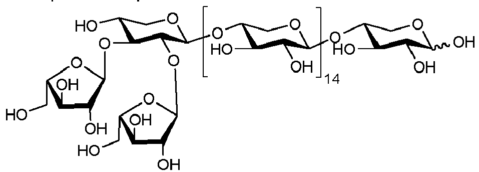 | 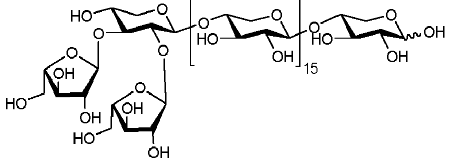 |
| **85:** (A²⁺³X₁₇) | **86:** (A²⁺³X₁₈) |
| 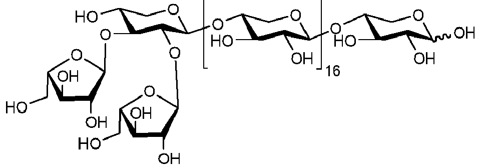 | 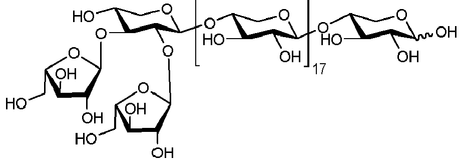 |
| **87:** (A²⁺³X₁₉) | **88:** (A²⁺³X₂₀) |
| 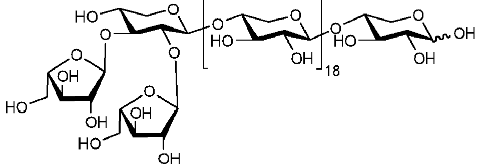 | 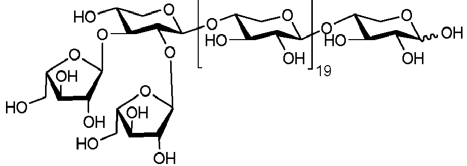 |
| **89:** (XA²X₇) | **90:** (XA²X₈) |
| 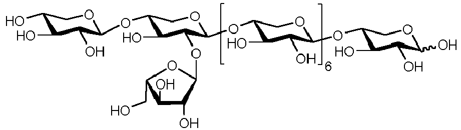 | 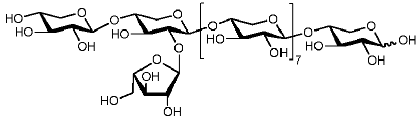 |
| **91:** (XA²X₉) | **92:** (XA²X₁₀) |
| 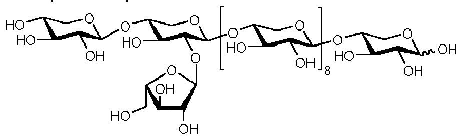 | 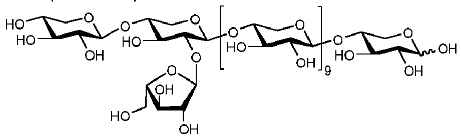 |
| **93:** (XA²X₁₁) | **94:** (XA²X₁₂) |
| 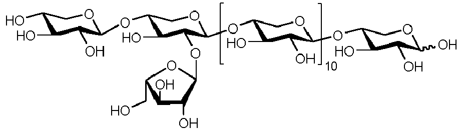 | 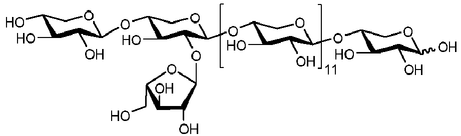 |
| **95:** (XA²X₁₃) | **96:** (A²X₁₄) |
| 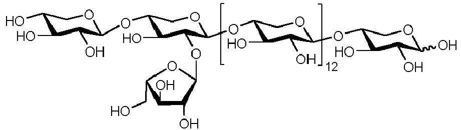 | 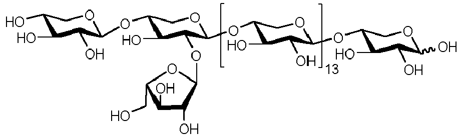 |
| **97:** (XA²X₁₅) | **98:** (XA²X₁₆) |
| 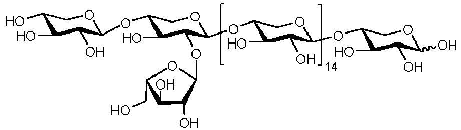 | 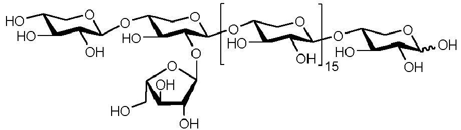 |
| **99:** (XA²X₁₇) | **100:** (XA²X₁₈) |
| 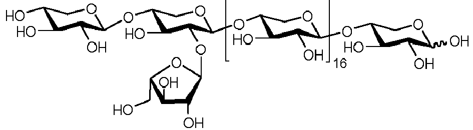 | 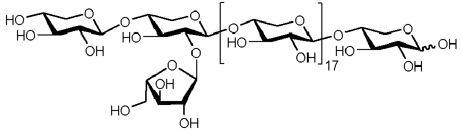 |
| **101:** (XA²X₁₉) | **102:** (XA²X₂₀) |
| 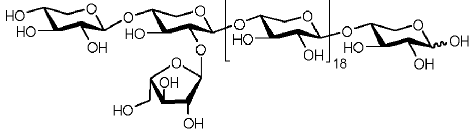 | 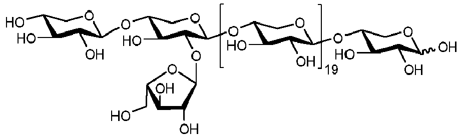 |
| **103:** (XA³X₇) | **104:** (XA³X₈) |
| 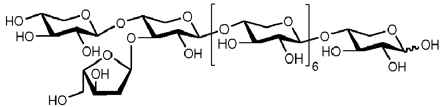 | 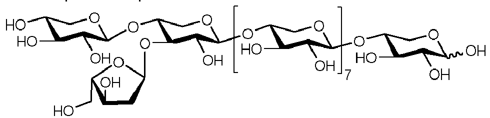 |
| **105:** (XA³X₉) | **106:** (XA³X₁₀) |
| 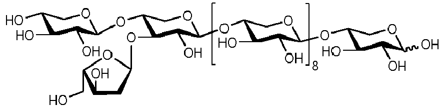 | 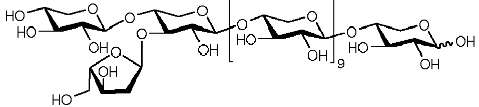 |
| **107:** (XA³X₁₁) | **108:** (XA³X₁₂) |
| 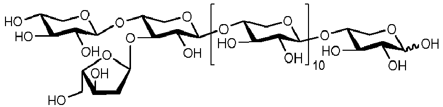 | 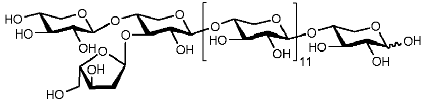 |
| **109:** (XA³X₁₃) | **110:** (XA³X₁₄) |
| 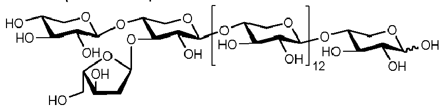 | 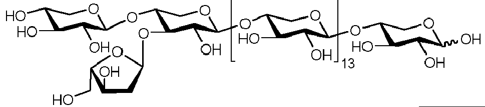 |
| **111:** (XA³X₁₅) | **112:** (XA³X₁₆) |
| 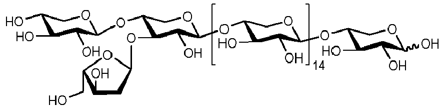 | 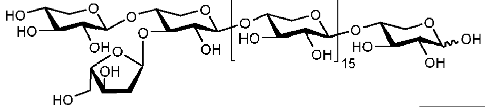 |
| **113:** (XA³X₁₇) | **114:** (XA³X₁₈) |
| 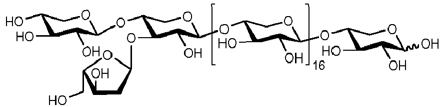 | 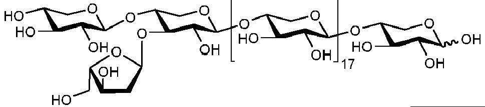 |
| **115:** (XA³X₁₉) | **116:** (XA³X₂₀) |
| 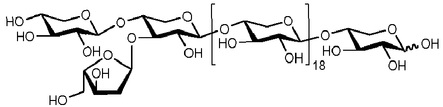 | 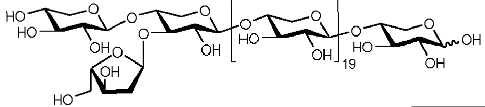 |
| **117:** (XA²⁺³X₇) | **118:** (XA²⁺³X₈) |
| 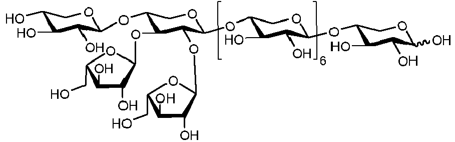 | 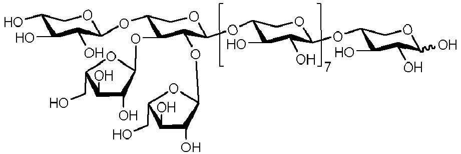 |
| **119:** (XA²⁺³X₈) | **120:** (XA²⁺³X₁₀) |
| 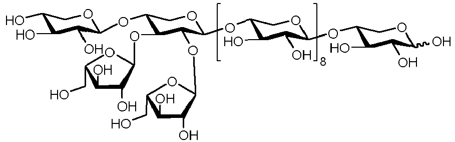 | 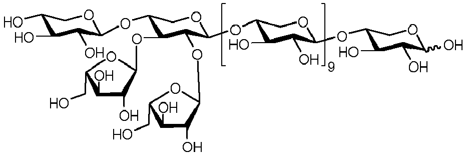 |
| **121:** (XA²⁺³X₁₁) | **122:** (XA²⁺³X₁₂) |
| 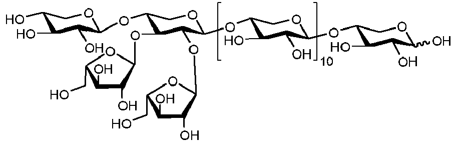 | 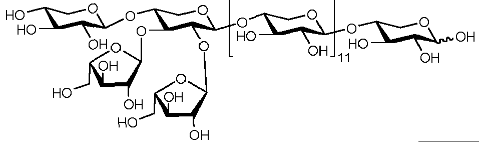 |
| **123:** (XA²⁺³X₁₃) | **124:** (XA²⁺³X₁₄) |
| 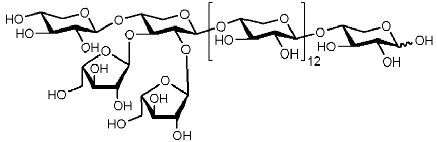 | 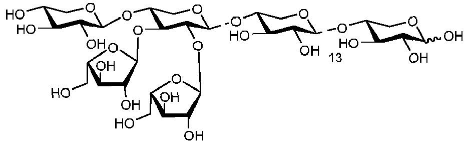 |
| **125:** (XA²⁺³X₈) | **126:** (XA²⁺³X₈) |
| 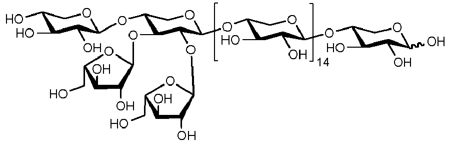 | 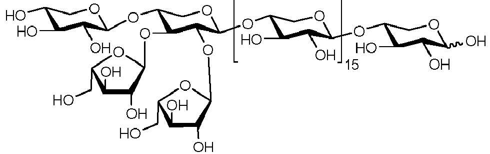 |
| **127:** (XA²⁺³X₁₇) | **128:** (XA²⁺³X₁₈) |
| 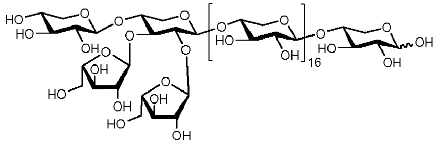 | 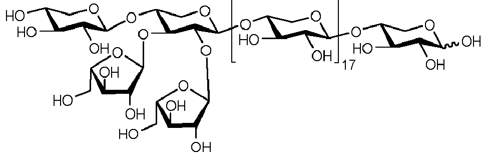 |
| **129:** (XA²⁺³X₁₉) | **130:** (XA²⁺³X₂₀) |
| 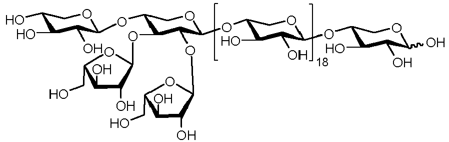 | 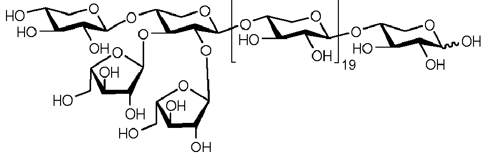 |

### Abbreviations

- *L*-Ara*f* :: L-arabinofuranosyl unit
- α-*L*-Ara*f* :: α-L-arabinofuranoside
- *D*-Xyl*p* :: D-xylopyranosyl unit
- **AXOS** :: arabinoxylo-oligosaccharides
- **XOS** :: xylo-oligosaccharides

- **A² :**: α-L-Ara*f*-(1,2)-β-D-Xyl*p*
- **A³** :: α-L-Ara*f*-(1,3)-β-D-Xyl*p*
- **A²⁺³** :: [α-L-Ara*f*-(1,2)][α-L-Ara*f*-(1,3)]-β-D-Xyl*p*
- **X** :: *D*-xylose
- **X₂** :: (1,4)-β-D-xylobiose
- **X₃** :: (1,4)-β-D-xylotriose
- **X₄** :: (1,4)-β-D-xylotetraose
- **X₅** :: (1,4)-β-D-xylopentaose
- **X₆** :: (1,4)-β-D-xylohexaose

- **A²X** :: α-L-Ara*f*-(1,2)-β-D-Xyl*p*-(1,4)-β-D-Xyl.
- **A²X₂** :: α-L-Ara*f*-(1,2)-β-D-Xyl*p*-(1,4)-β-D-Xyl*p*-(1,4)-D-Xyl.
- **A²X₃** :: α-L-Ara*f*-(1,2)-β-D-Xyl*p*-(1,4)-β-D-Xyl*p*-(1,4)-β-D-Xyl*p*-(1,4)-D-Xyl
- **A²X₄** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-D-Xyl
- **A²X₅** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₆** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₇** :: α-L-Ara*f*-(1,2)-β-D-Xyl*p*-(1,4)-β-D-Xyl*p*-(1,4)-β**-**D-Xyl*p*-(1,4)-β-D-Xyl*p*-(1,4)-β**-**D-Xyl*p*-(1,4)-β-D-Xyl*p*-(1,4)-β-D-Xyl*p*-(1,4)-D-Xyl
- **A²X₈** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₉** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₁₀** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl*p-*(1,4)-D-Xyl
- **A²X₁₁** :: α-L-Ara*f*-(12)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **A²X₁₂** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **A²X₁₃** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **A²X₁₄** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₁₅** :: α-L-Ara*f*-(12)-β-D-Xyl*p*-(1,4)-β-D-Xyl*p*-(1,4)-β-D-Xyl*p*-(1,4)-β-D-Xyl*p*-(1,4)-β**-**D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **A²X₁₆** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **A²X₁₇** :: α-L-Ara*f*-(1,2)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **A²X₁₈** :: α-L-Ara*f*-(12)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **A²X₁₉** :: α-L-Ara*f*-(1,2)-β-D-Xyl*p*-(1,4)-β-D-Xyl*p*-(1,4)-p-β-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl
- **A²X₂₀** :: α-L-Ara*f*-(12)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl

- **A³X** :: α-L-Ara*f*-(1,3)-β-D-Xyl*p*-(1,4)-β-D-Xyl.
- **A³X₂** :: α-L-Ara*f*-(1,3)-β-D-Xyl*p*-(1,4)-β-D-Xyl*p*-(1,4)-D-Xyl.
- **A³X₃** :: α-L-Ara*f*-(1,3)-β-D-Xyl*p*-(1,4)-β-D-Xyl*p*-(1,4)-β-D-Xyl*p*-(1,4)-D-Xyl
- **A³X₄** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-D-Xyl
- **A³X₅** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xylp-(1,4)-D-Xyl
- **A³X₆** :: α-L-Ara*f*-(1,3)-β-D-Xyl*p*-(1,4)-β-D-Xyl*p*-(1,4)-β-D-Xyl*p*-(1,4)-β**-**D-Xyl*p*-(1,4)-β-D-Xyl*p*-(1,4)-β-D-Xyl*p*-(1,4)-D-Xyl
- **A³X₇** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A³X₈** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A³X₉** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A³X₁₀** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **A³X₁₁** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **A³X₁₂** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **A³X₁₃** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **A³X₁₄** :: α-L-Ara*f*-1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl
- **A³X₁₅** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **A³X₁₆** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **A³X₁₇** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **A³X₁₈** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β**-**D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **A³X₁₉** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl
- **A³X₂₀** :: α-L-Ara*f*-(1,3)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β**-**D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl

- **A²⁺³X** :: [α-L-Ara*f*-(1,2)][α-L-Ara*f*-(1,3)]-β-D-Xyl*p*-(1,4)-β-D-Xyl
- **A²⁺³X₂** :: [α-L-Ara*f*-(1,2)][α-L-Ara*f*-(1,3)]-β-D-Xyl*p*-(1,4)-β-D-Xyl*p*-(1,4)-D-Xyl
- **A²⁺³X₃** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-D-Xyl
- **A²⁺³X₄** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₅** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₆** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₇** :: [α-L-Ara*f*-(1,2)][α-L-Ara*f-*(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₈** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₉** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₀** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-P-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₁** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₂** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₃** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β*-*D-Xyl*p*-(1,4)-β-D-Xyl*p*-(1,4)-β-D-Xyl*p*-(1,4)-β-D-Xyl*p*-(1,4)-D-Xyl
- **A²⁺³X₁₄** :: [α-L-Ara*f*-(1,2)][α-L-Araf-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₅** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₆** :: [α-L-Ara*f*-(1,2)][α-L-Ara*f*-(1,3)]-β**-**D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₇** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₈** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₁₉** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **A²⁺³X₂₀** :: [α-L-Ara*f*-(1,2)][α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl

- **XA²X** :: β-D-Xyl*p*-(1,4)-[α-L-Ara*f*-(1,2)]-β-D-Xyl*p*-(1,4)-D-Xyl.
- **XA²X₂** :: β-D-Xyl*p*-(1,4)-[α-L-Ara*f*-(1,2)]-β-D-Xyl*p*-(1,4)-β-D-Xyl*p*-(1,4)-D-Xyl.
- **XA²X₃** :: β-D-Xyl*p*-(1,4)-[α-L-Ara*f*-(1,2)]-β-D-Xyl*p*-(1,4)-β-D-Xyl*p*-(14)-β-D-Xyl*p*-(1,4)-D-Xyl.
- **XA²X₄** :: β-D-Xyl*p*-(1,4)-[α-L-Ara*f*-(1,2)]-β-D-Xyl*p*-(1,4)-β**-**D-Xyl*p*-(1,4)-β-D-Xyl*p*-(1,4)-β-D-Xyl*p*-(1,4)-D-Xyl.
- **XA²X₅** :: β-D-Xyl*p*-(1,4)-[α-L-Ara*f*-(1,2)]-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl.
- **XA²X₆** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl.
- **XA²X₇** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²X₈** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β*-*D-Xyl*p*-(1,4)-β-D-Xyl*p*-(1,4)-β-D-Xyl*p*-(1,4)-β-D-Xyl*p*-(1,4)-D-Xyl
- **XA²X₉** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xylp*-*(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²X₁₀** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl*p*-(1,4)-D-Xyl
- **XA²X₁₁** :: β-D-Xyl*p*-(14)-[α-L-Ara*f*-(1,2)]-β-D-Xyl*p*-(14)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²X₁₂** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²X₁₃** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²X₁₄** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-*p*-D-Xyl*p*-(1,4)-β-D-Xyl*p*-(1,4)-β-D-Xylp-(1,4)-D-Xyl
- **XA²X₁₅** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **XA²X₁₆** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **XA²X₁₇** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **XA²X₁₈** :: β-D-Xyl*p*-(1,4)-[α-L-Ara*f*-(1,2)]-β-D-Xyl*p*-(1,4)-β-D-Xyl*p*-(14)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xylp-(1,4)-D-Xyl
- **XA²X₁₉** :: β-D-Xyl*p*-(1,4)-[α-L-Ara*f*-(1,2)]-β-D-Xyl*p*-(1,4)-β-D-Xyl*p*-(1,4)-β-D-Xylβ-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA²X₂₀** :: β-D-Xyl*p*-(1,4)-[α-L-Ara*f*-(1,2)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl

- **XA³X** :: β-D-Xyl*p*-(1,4)-[α-L-Ara*f*-(1,3)]-β-D-Xyl*p*-(1,4)-D-Xyl.
- **XA³X₂ :**: β-D-Xyl*p*-(1,4)-[α-L-Ara*f*-(1,3)]-β-D-Xyl*p*-(1,4)-β-D-Xyl*p*-(1,4)-D-Xyl.
- **XA³X₃** :: β-D-Xyl*p*-(1,4)-[α-L-Ara*f*-(1,3)]-β-D-Xyl*p*-(1,4)-β-D-Xyl*p*-(1,4)-β**-**D-Xyl*p*-(1,4)-D-Xyl.
- **XA³X₄** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-D-Xyl.
- **XA³X₅** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl.
- **XA³X₆** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl.
- **XA³X₇** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₈** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β*-*D-Xyl*p*-(1,4)-β-D-Xyl*p*-(1,4)-β-D-Xyl*p*-(1,4)-β-D-Xyl*p*-(1,4)-D-Xyl
- **XA³X₉** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₁₀** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₁₁** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₁₂** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₁₃** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-D-Xyl
- **XA³X₁₄** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA³X₁₅** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **XA³X₁₆** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **XA³X₁₇** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **XA³X₁₈** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xylp-(1,4)-D-Xyl
- **XA³X₁₉** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA³X₂₀** :: β-D-Xyl*p*-(1,4)-[α-L-Ara*f*-(1,3)]-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl

- **XA²⁺³X** :: β-D-Xyl*p*-(1,4)-[α-L-Ara*f*-(1,2)][α-L-Ara*f*-(1,3)]-β-D-Xyl*p*-(1,4)-β-D-Xyl.
- **XA²⁺³X₂** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-D-Xyl.
- **XA²⁺³X₃** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-D-Xyl.
- **XA²⁺³X₄** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-D-Xyl.
- **XA²⁺³X₅** :: β-D-Xyl*p*-(1,4)-[β-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl.
- **XA²⁺³X₆** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl.
- **XA²⁺³X₇** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA²⁺³X₈** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **XXA²⁺³X₉** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **XA²⁺³X₁₀** :: β-D-Xyl*p*-(1,4)-[α-L-Ara*f*-(1,2)][α-L-Ara*f*-(1,3)]-β**-**D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **XA²⁺³X₁₁** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-D-Xyl
- **XA²⁺³X₁₂** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA²⁺³X₁₃** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-D-Xyl
- **XA²⁺³X₁₄** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β**-**D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **XA²⁺³X₁₅** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β**-**D-Xyl*p*-(1,4)-β-D-Xyl*p*-(1,4)-β-D-Xyl*p-*(1,4)-β-D-Xyl*p-*(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl
- **XA²⁺³X₁₆** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xylp-(1,4)-D-Xyl
- **XA²⁺³X₁₇** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-D-Xyl
- **XA²⁺³X₁₈** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-p-D-Xyl*p-*(1,4)-β-D-Xyl*p*-(1,4)-D-Xyl
- **XA²⁺³X₁₉** :: β-D-Xyl*p*-(1,4)-[α-L-Ara*f*-(1,2)][α-L-Ara*f*-(1,3)]-β-D-Xyl*p*-(1,4)-β**-**D-Xyl*p-*(1,4)-β-D-Xyl*p-*(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-D-Xyl
- **XA²⁺³X₂₀** :: β-D-Xyl*p*-(1,4)-[α-L-Ara*f*-(1,2)][α-L-Ara*f*-(1,3)]-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-β-D-Xyl*p*-(1,4)-β-D-Xyl*p-*(1,4)-β-D-Xyl*p*-(1,4)-β-D-Xyl*p*-(1,4)-β-D-Xyl*p*-(1,4)-D-Xyl.

Thus, in some aspect, the present invention is directed to a method for producing xylose oligosaccharides comprising:
performing transglycosylation reaction of a glycosyl donor with a glycosyl acceptor by using the polypeptide of the invention,
   wherein
   the glycosyl donor is selected from the group consisting of: xylose, xylobiose, xylotriose, xylotetraose, xylopentaose, xylohexaose and xyloheptaose; and
   the glycosyl acceptor is a substrate containing at least 2 xylose units; and
   the substrate containing at least 2 xylose units has the formula (II): 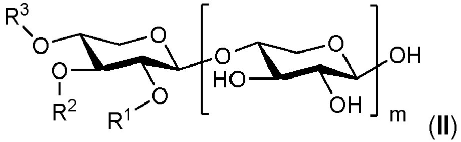 wherein,
      **m** is an integer selected from 1 to 6;
         preferably, 2 to 5, more preferably 3 to 5;
      **R¹** represents-H, or 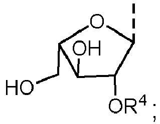
      **R²** represents -H, or 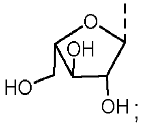
      **R³** represents -H, or 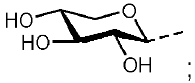
      **R⁴** represents -H;
      and **R¹** and **R²** are not -H at the same time.

Preferably, the glycosyl donors is selected from xylotriose (**3**), xylotetraose (**4**), xylopentaose (**5**), and xylohexaose (**6**).

Preferably, in said method as described herein, each xylose oligosaccharide has the formula (I): 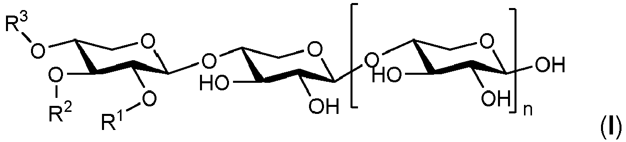 wherein
n is an integer selected from 1 to18;
   preferably, 1 to 16, more preferably 1 to 14;
   still more preferably, 1 to 12, most preferably 1 to 10;
**R¹** represents-H, or 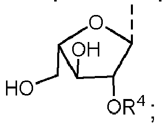
**R²** represents -H, or 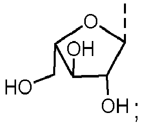
**R³** represents -H, or 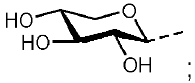
**R⁴** represents -H;
and **R¹** and **R²** are not -H at the same time.

### Alternative method for producing arabinoxylan oligosaccharides (AXOS)

In one aspect, the present invention relates to a method for producing xylose oligosaccharides comprising:
1) performing transglycosylation reaction of a glycosyl donor with a glycosyl acceptor by using the polypeptide of the invention,
   wherein
   the glycosyl donor is selected from the group consisting of: xylose, xylobiose, xylotriose, xylotetraose, xylopentaose, xylohexaose and xyloheptaose; and
   the glycosyl acceptor is a substrate containing at least 2 xylose units, and the substrate containing at least 2 xylose units has the formula (II): 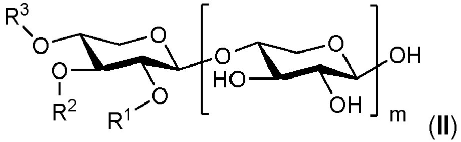 wherein,
      **m** is an integer selected from 1 to 6;
         preferably, 2 to 5, more preferably 3 to 5;
      **R¹** represents-H;
      **R²** represents -H; 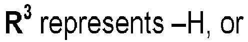 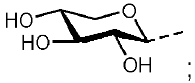
      **R⁴** represents -H;
2) performing transglycosylation reaction of an L-arabinose donor with the resulting xylooligosaccharide obtained after the step 1) by using an α-_{L}-arabinofuranosidase (E.C. 3.2.1.55).

Preferably, in said method as described herein, the glycosyl donor is selected from the group consisting of: xylotriose (**3**), xylotetraose (**4**), xylopentaose (**5**), xylohexaose (**6**), and xyloheptaose (**7**), more preferably, xylotriose (**3**), xylotetraose (**4**), xylopentaose (**5**), xylohexaose (**6**), and xyloheptaose (**7**); still more preferred, xylotetraose (**4**), xylopentaose (**5**), and xylohexaose (**6**).

The L-arabinose donor has a formula (**A***) 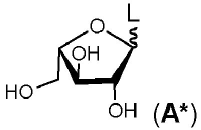 wherein **L** is an activating group selected from; -OH, -F, and -O-(4-NO₂-Ph).

Said α-_{L}-arabinofuranosidase (E.C. 3.2.1.55) forms at least one of α-L-Ara*f-*(1,2)-or α-L-Ara*f*-(1,3)]- moiety with (β-D-Xyl*p*-(1,4) of the xylooligosaccharides obtained after the step 1).

Thus, after the step 2), the each xylose oligosaccharide as obtained has the formula (I): 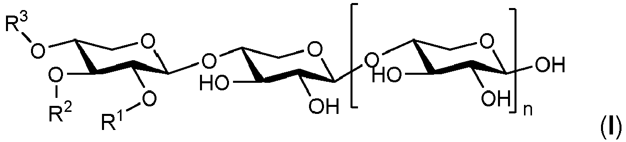 wherein
n is an integer selected from 1 to18;
**R¹** represents-H, or 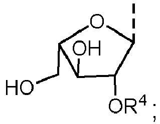
**R²** represents -H, or 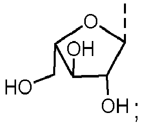
**R³** represents -H, or 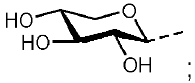
**R⁴** represents -H, or

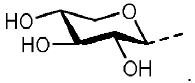

In some embodiments, the present invention relates to a mixture of xylose oligosaccharides obtainable by said method as described herein. Preferably, the xylose oligosaccharides xylose oligosaccharides obtainable by said method are xylooligosaccharides (XOS), arabinoxylan oligosaccharides (AXOS), or fragments thereof.

Each xylose oligosaccharide xylose oligosaccharides obtainable by said method has the formula (I): 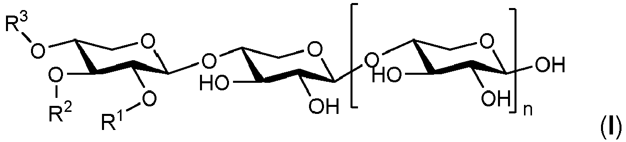 wherein
n is an integer selected from 1 to18;
**R¹** represents-H, or 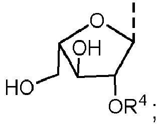
**R²** represents -H, or 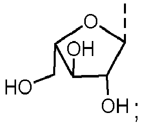
**R³** represents -H, or 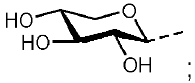
**R⁴** represents -H, or

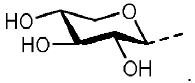

Preferably, the mixture of xylose oligosaccharides obtainable by said method as described above, comprises two or more xylose oligosaccharides, wherein xylose oligosaccharides are xylooligosaccharides (XOS) , arabinoxylan oligosaccharides (XOS), or fragments thereof:

In some aspects, the mixture of xylose oligosaccharides obtainable by the method of the present invention, comprise or consisting of: compounds **3** - **20** and/or **23** -**130**:

Preferably, the mixture of xylose oligosaccharides obtainable by the method of the present invention, comprise or consisting of: compounds **3** - **20**, when the glycosyl acceptor is a substrate containing at least 2 xylose units, and
the substrate containing at least 2 xylose units has the formula (II): 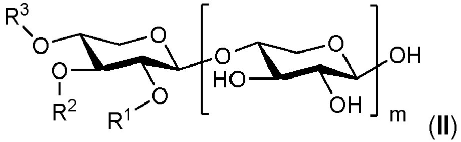 wherein,
**m** is an integer selected from 1 to 6;
   preferably, 2 to 5, more preferably 3 to 5;
**R¹** represents-H;
**R²** represents -H;
**R³** represents -H, or 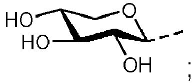
**R⁴** represents -H.

Preferably, the mixture of xylose oligosaccharides obtainable by the method of the present invention, comprise or consisting of: compounds **23 -130,** when the glycosyl acceptor is a substrate containing at least 2 xylose units, and
the substrate containing at least 2 xylose units has the formula (II): 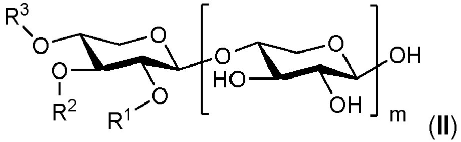 wherein,
**m** is an integer selected from 1 to 6;
   preferably, 2 to 5, more preferably 3 to 5;
**R¹** represents-H, or 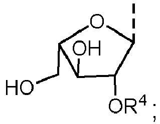
**R²** represents -H, or 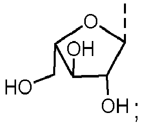
**R³** represents -H, or 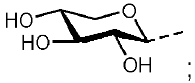
**R⁴** represents -H;
and **R¹** and **R²** are not -H at the same time.

In some aspects, the present invention refers to a mixture of xylose oligosaccharides obtainable by the preparation method of the present invention as described above, comprising or consisting of: xylooligosaccharides (XOS), and/or arabinoxylan oligosaccharides (AXOS), preferably xylooligosaccharides (XOS) **3** - **20**, and/or arabinoxylan oligosaccharides (AXOS) **23** - **130**.

In some embodiments, the mixture of xylose oligosaccharides obtainable by the method of the present invention, comprises or consists of: compounds **3** - **20**, i.e. xylotriose (**3**), xylotetraose (**4**), xylopentaose (**5**), xylohexaose (**6**), xyloheptaose (**7**), xylooctaose (**8**), xylononaose (**9**), xylodecaose (**10**), xyloundecaose (**11**), xylododecaose (**12**), xylotridecaose (**13**), xylotetradecaose (**14**), xylopentadecaose (**15**), xylohexadecaose (**16**), xyloheptadecaose (**17**), xylooctadecaose (**18**), xylononadecaose (**19**), and xyloicosaose (**20**).

Preferably, the mixture of xylose oligosaccharides obtainable by the method of the present invention, comprises or consists of: xylotriose (**3**), xylotetraose (**4**), xylopentaose (**5**), xylohexaose (**6**), xyloheptaose (**7**), xylooctaose (**8**), xylononaose (**9**), xylodecaose (**10**), xyloundecaose (**11**), xylododecaose (**12**), xylotridecaose (**13**), xylotetradecaose (**14**), and xylopentadecaose (**15**).

More preferably, the mixture of xylose oligosaccharides obtainable by the method of the present invention, consists of: xylotriose (**3**), xylotetraose (**4**), xylopentaose (**5**), xylohexaose (**6**), xyloheptaose (**7**), xylooctaose (**8**), xylononaose (**9**), and xylodecaose (**10**).

In some embodiments, the mixture of xylose oligosaccharides obtainable by the method of the present invention, comprises or consists of: compounds **23** - **130**, i.e., arabinoxylan oligosaccharides (AXOS) **23** - **130**.

Preferably, the mixture of xylose oligosaccharides obtainable by the method of the present invention, comprises or consists of: arabinoxylan oligosaccharides (AXOS) **23** - **55**, **61** - **69**, **75** - **83, 89** - **97**, **103-111,** and/or **117 - 125.**

More preferably, the mixture of xylose oligosaccharides obtainable by the method of the present invention, comprises or consists of: arabinoxylan oligosaccharides (AXOS) **23** - **50, 61** - **64, 75** - **78**, **89** - **92, 103 - 106,** and/or **117 - 120.**

Still more preferably, the mixture of xylose oligosaccharides obtainable by the method of the present invention, comprises or consists of: arabinoxylan oligosaccharides (AXOS) **23** - **46**.

Preferably, the mixture of xylose oligosaccharides obtainable by the method of the present invention, comprises or consists of: arabinoxylan oligosaccharides (AXOS), **A²X₃, A²X₄, A²X₅, A²X₆, A²X₇, A²X₈, A²X₉, A²X₁₀, A²X₁₁, A²X₁₂, A²X₁₃, A²X₁₄,** and **A²X₁₅.**

Still more preferably, the mixture of xylose oligosaccharides obtainable by the method of the present invention, comprises or consists of: arabinoxylan oligosaccharides (AXOS), **A²X₃, A²X₄, A²X₅, A²X₆, A²X₇, A²X₈, A²X₉,** and **A²X₁₀.**

Preferably, the mixture of xylose oligosaccharides obtainable by the method of the present invention, comprises or consists of: arabinoxylan oligosaccharides (AXOS), **A³X₃, A³X₄, A³X₅, A³X₆, A³X₇, A³X₈, A³X₉, A³X₁₀, A³X₁₁, A³X₁₂, A³X₁₃, A³X₁₄,** and **A³X₁₅.**

Still more preferably, the mixture of xylose oligosaccharides obtainable by the method of the present invention, comprises or consists of: arabinoxylan oligosaccharides (AXOS), **A³X₃, A³X₄, A³X₅, A³X₆, A³X₇, A³X₈, A³X₉,** and **A³X₁₀.**

Preferably, the mixture of xylose oligosaccharides obtainable by the method of the present invention, comprises or consists of: arabinoxylan oligosaccharides (AXOS), **A²⁺³X₃, A²⁺³X₄, A²⁺³X₅, A²⁺³X₆, A²⁺³X₇, A²⁺³X₈, A²⁺³X₉, A²⁺³X₁₀, A²⁺³X₁₁, A²⁺³X₁₂, A²⁺³X₁₃, A²⁺³X₁₄,** and **A²⁺³X₁₅.**

Still more preferably, the mixture of xylose oligosaccharides obtainable by the method of the present invention, comprises or consists of: arabinoxylan oligosaccharides (AXOS), **A²⁺³X₃, A²⁺³X₄, A²⁺³X₅, A²⁺³X₆, A²⁺³X₇, A²⁺³X₈, A²⁺³X₉,** and **A²⁺³X₁₀.**

Preferably, the mixture of xylose oligosaccharides obtainable by the method of the present invention comprises or consists of: arabinoxylan oligosaccharides (AXOS), **XA²X₃, XA³X₃, XA²X₄, XA³X₄, XA²X₅, XA³X₅, XA²X₆, XA³X₆, XA²X₇, XA³X₇, XA²X₈, XA³X₈, XA²X₉, XA³X₉, XA²X₁₀, XA³X₁₀, XA²X₁₁, XA³X₁₁, XA²X₁₂, XA³X₁₂, XA²X₁₃, XA³X₁₃, XA²X₁₄, XA³X₁₄, XA²X₁₅** and **XA³X₁₅.**

Still more preferably, the mixture of xylose oligosaccharides obtainable by the method of the present invention comprises or consists of: arabinoxylan oligosaccharides (AXOS), **XA²X₃, XA³X₃, XA²X₄, XA³X₄, XA²X₅, XA³X₅, XA²X₆, XA³X₆, XA²X₇, XA³X₇, XA²X₈,XA³X₈, XA²X₉, XA³X₉, XA²X₁₀,** and **XA³X₁₀.**

Preferably, the mixture of xylose oligosaccharides obtainable by the method of the present invention comprises or consists of: arabinoxylan oligosaccharides (AXOS), **XA²X₃, XA²X₄, XA²X₅, XA²X₆, XA²X₇, XA²X₈,XA²X₉, XA²X₁₀, XA²X₁₁, XA²X₁₂, XA²X₁₃, XA²X₁₄,** and **XA²X₁₅.**

Still more preferably, the mixture of xylose oligosaccharides obtainable by the method of the present invention comprises or consists of: arabinoxylan oligosaccharides (AXOS), **XA²X₃, XA²X₄, XA²X₅, XA²X₆, XA²X₇, XA²X₈, XA²X₉,** and **XA²X₁₀.**

Preferably, the mixture of xylose oligosaccharides obtainable by the method of the present invention, comprises or consists of: arabinoxylan oligosaccharides (AXOS), **XA³X₃, XA³X₄, XA³X₅, XA³X₆, XA³X₇, XA³X₈, XA³X₉, XA³X₁₀, XA³X₁₁, XA³X₁₂, XA³X₁₃, XA³X₁₄,** and **XA³X₁₅.**

Still more preferably, the mixture of xylose oligosaccharides obtainable by the method of the present invention comprises or consists of: arabinoxylan oligosaccharides (AXOS), **XA³X₃, XA³X₄, XA³X₅, XA³X₆, XA³X₇, XA³X₈, XA³X₉,** and **XA³X₁₀.**

Preferably, the mixture of xylose oligosaccharides obtainable by the method of the present invention, comprises or consists of: arabinoxylan oligosaccharides (AXOS), **XA²⁺³X₃, XA²⁺³X₄, XA²⁺³X₅, XA²⁺³X₆, XA²⁺³X₇, XA²⁺³X₈, XA²⁺³X₉, XA²⁺³X₁₀, XA²⁺³X₁₁, XA²⁺³X₁₂, XA²⁺³X₁₃, XA²⁺³X₁₄,** and **XA²⁺³X₁₅.**

Still more preferably, the mixture of xylose oligosaccharides obtainable by the method of the present invention, comprises or consists of: arabinoxylan oligosaccharides (AXOS), **XA²⁺³X₃, XA²⁺³X₄, XA²⁺³X₅, XA²⁺³X₆, XA²⁺³X₇, XA²⁺³X₈, XA²⁺³X₉,** and **XA²⁺³X₁₀.**

In another aspect, the present invention is directed to use of said mixture of xylose oligosaccharides obtained or obtainable by the preparation method of the invention as described above, comprising or consisting of: xylooligosaccharides (XOS), and/or arabinoxylan oligosaccharides (AXOS), preferably xylooligosaccharides (XOS) 3 - **20,** and/or arabinoxylan oligosaccharides (AXOS) **23** - **130**.

In some aspects, said mixture of xylose oligosaccharides is useful in life science, preferably, said mixture of xylose oligosaccharides is useful in chemical analytics and enzymology. In case of use in chemical analytics, said mixture of xylose oligosaccharides is use as oligosaccharide standards. In case of use in said mixture of xylose oligosaccharides is for use in chemical analytics, said mixture of xylose oligosaccharides is use as potential substrates for the characterization of xylan-degrading enzymes.

In some embodiments, said mixture of xylose oligosaccharides is useful as prebiotics, nutraceuticals, pharmaceutically active ingredients, or food supplements.

In some embodiments, said mixture of xylose oligosaccharides is useful as pharmaceutically active ingredients in the prophylaxis and/or treatment of bacterial infectious diseases, cancers, immune diseases, type 2 diabetes, or cardiovascular diseases.

The mixtures of the xylose oligosaccharides (also named xylooligosaccharides) are especially useful for promoting gastrointestinal regularity, for regeneration of the intestinal flora, for improving glycemic control, and for the treatment of diarrhea and constipation. Moreover, such xylose oligosaccharide mixtures are useful for controlling or reducing the blood cholesterol level and for maintaining healthy blood sugar levels. So far, undesired side effect could not be observed even at higher XOS dosages.

### Description of Figures

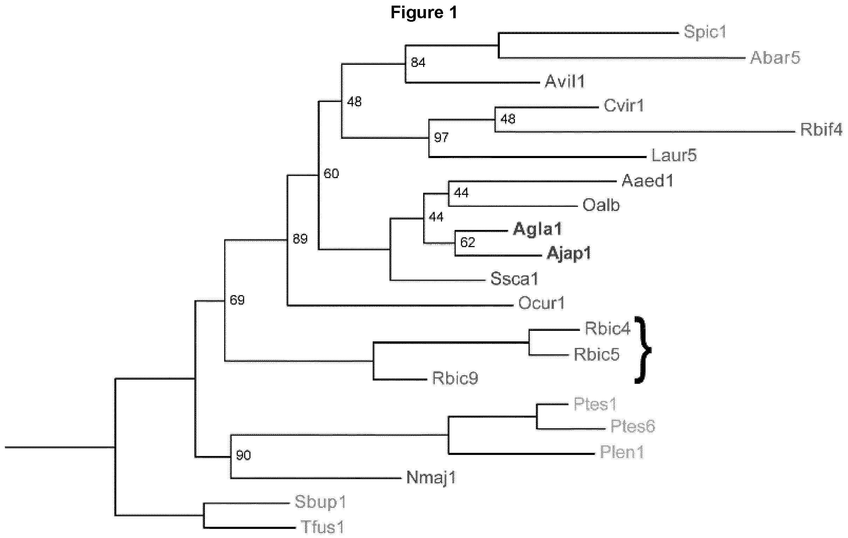

**Figure 1****:** Phylogenetic relationships of *R. bicolor* Rbic4, 5 and 9 with counterparts from other species of long-horned beetles. The functionally characterized xylanases Agla1 (XP_018565006.1) and Ajap1 (AHI15746.1) are indicated in bold. *Rbic4, 5 and 9 originate from species-specific recent gene duplications.*
**Figure 2****:** Enzyme activity of recombinant Rbic4, Rbic5 and Rbic9 towards polysaccharides of the plant cell wall and xylan oligosaccharides. Recombinant proteins were expressed in Sf9 insect cells, bound to anti-V5 agarose beads and incubated with polysaccharides found in plant cell walls, as well as with xylan oligosaccharides (xylotriose (X3) to xylohexaose (X6)) for 16 h in 50 mM Mclllvaine buffer pH 5.0 at 40°C. The resulting enzyme assays were then spotted on a thin layer chromatography (TLC) plate. Substrate used are indicated at the bottom of each TLC plate. Xylose (X1) to xylohexaose (X6) were used as standards.
**Figure 3****:** Influence of the ratio enzyme/substrate on the enzymatic activity of Rbic4, Rbic5 and Rbic9. Enzyme assays were set up with a fixed amount of substrate, 0.2 percent for xylan polysaccharide and 5 µg for xylohexaose. Increasing amount of enzyme were used (for details regarding what 1x of enzyme corresponds to, please refer below to the method description of the enzyme assays). Xylose (X1) to xylohexaose (X6) were used as standards.
**Figure 4****:** Time-course assay of recombinant Rbic4 incubated with four xylooligosaccharides of different size as substrates. Recombinant Rbic4 was expressed in Sf9 insect cells, bound to anti-V5 agarose beads and incubated with either xylotriose, xylotetraose, xylopentaose or xylohexaose at various times ranging from 10 min to 8 h. Enzyme assays were run in 50 mM Mclllvaine buffer pH 5.0 at 40°C. Resulting products generated by Rbic4 on the substrates tested were visualized by thin layer chromatography (TLC). The accumulation of long xylooligosaccharides can be observed already after 10 min incubation, except when xylotriose was used as a substrate. Standards used: xylose (X1) to xylohexaose (X6). Controls correspond to the substrate incubated without enzyme.

### Examples

### Example 1: Comparison of the enzymatic activity of Rbic4 to the one of the closely similar Rbic5 and Rbic9

To test whether Rbic4, 5 and 9 are endo-β-1,4-xylanases as expected, we first attempted to amplify their cDNA by RT-PCR, which worked fine for Rbic4 and Rbic9, but not for Rbic5. In fact, the sequences corresponding to Rbic4 and Rbic5 are so similar that discriminating PCR primers were difficult to design. We thus decided to get Rbic5 gene-synthesized, codon-optimized and cloned into an expression vector compatible with Insect Sf9 cells. Similarly, PCR-amplified Rbic4 and Rbic9 were also cloned into an expression vector compatible with insect Sf9 cells. To detect positive expression of these constructs by Sf9 cells, a V5-(His)₆ epitope was cloned in frame with Rbic4, 5 and 9 at the carboxyl-terminus of each protein. This epitope allows detection of the expressed proteins by Western blot using an anti-V5-HRP antibody. Although Rbic4 could be expressed by Sf9 cells without much trouble, the Rbic9 construct did not lead to any detectable recombinant proteins after transfection in Sf9 cells. We thus decided to get Rbic9 gene-synthesized with codon optimisation for expression in Sf9 cells. This last Rbic9 construct could be expressed without any problem by Sf9 cells. All three recombinant proteins were located in the culture medium of Sf9 cells because they naturally harbour an amino-terminal secretion signal (signal peptide).

### 1.1 Summary of the constructs that led to positive heterologous expression in Sf9 cells:

- PCR-amplified Rbic4 cloned into pIB/V5-His TOPO/TA vector (Invitrogen), in frame with a V5-(His)₆ epitope at the carboxyl-terminus.
- Gene-synthesized, codon-optimized Rbic5 cloned into pIZ/V5-His vector (Invitrogen), in frame with a V5-(His)₆ epitope at the carboxyl-terminus.
- Gene-synthesized, codon-optimized Rbic9 cloned into pIZ/V5-His vector (Invitrogen), in frame with a V5-(His)₆ epitope at the carboxyl-terminus.

### 1.2 Transfection of the above described constructs and pull-down of the corresponding recombinant proteins:

Sf9 cells, cultured in SF-900 II serum-free medium (Gibco, Paisley, UK), were seeded in 6-well plates such that they covered 60 percent of the bottom of each well.

These cells were then transfected with 2.4 µg of the constructs described above, using FUGENE HD (Promega, Madison, WI, USA) as the transfection reagent. A mock transfection in which the plasmid DNA was omitted was performed and used as a negative control. After 72 h, the culture medium, which contained the heterologously expressed proteins, was harvested and centrifuged (16000xg, 5 min, 4 °C) in order to remove floating cells and cell debris. Culture medium corresponding to cells transfected with the three constructs were incubated with 15 µl of agarose beads coated with the anti-V5 antibody (Bethyl laboratories, 50 percent gel slurry), for 16 h at 4 °C. Beads harbouring the recombinant proteins at their surface were pulled down by centrifugation (1000xg, 2 min, 4 °C) and were washed twice in 150 µl of 10 mM Tris-HCI pH 7.5, 150 mM NaCl, 0.5 mM EDTA buffer. The beads were then resuspended in 75 µl of double-distilled water before activity assays. Positive heterologous expression was verified by Western blot using an anti-V5-HRP antibody.

### 1.3 Enzyme assays using the recombinant Rbic4, Rbic5 and Rbic9:

Enzyme assays of a total volume of 20 µl were set up using 7 µl (1x) of recombinant enzyme bound to anti-V5 agarose beads (from the 75 µl described above) mixed with 4 µl of a one percent solution (w/v) of substrate in a 20 mM citrate/phosphate buffer pH 5.0. The substrates used were carboxymethylcellulose (Sigma-Aldrich, Saint-Louis, MI, USA), regenerated amorphous cellulose (RAC), xyloglucan from Tamarind seeds (Megazyme, Bray, Ireland), glucomannan from konjac (Megazyme, Bray, Ireland), galactomannan from carob (Megazyme, Bray, Ireland), beechwood xylan (Sigma-Aldrich, Saint-Louis, MI, USA). The oligosaccharides used as standards or in enzyme assays were all purchased from Megazyme (Bray, Ireland). Enzyme assays using oligosaccharides as substrates were set up as follows: 7 µl of recombinant enzyme bound to anti-V5 agarose beads (from the 75 µl described above) were mixed with 0.5 µl of 10 µg/µl of a given oligosaccharide in a 20 mM citrate/phosphate buffer pH 5.0 for a total reaction volume of 20 µl. Enzyme assays were incubated for 16 h at 40 °C before being applied to TLC plates (silica gel 60, 20 x 20 cm, Merck, Darmstadt, Germany). Plates were developed with ethyl acetate/acetic acid/formic acid/water (9:3:1:4) as the mobile phase. Reaction end-products were then revealed by soaking the plates in 0.2 percent (w/v) orcinol in methanol/sulfuric acid (9:1), then heated briefly until spots appeared on the plates.

In order to pull down the heterologously expressed Rbic4, 5 and 9, the culture medium of Sf9 cells transfected with each of the three constructs above was harvested and incubated with agarose beads coated with the anti-V5 antibody. The resulting agarose beads now harbouring either Rbic4, Rbic5 or Rbic9 on their surface were incubated with polysaccharides generally found to be present in plant cell walls, such as cellulose, xyloglucan, xylan and mannans used as substrates. Xylan oligosaccharides were also tested as substrates (from xylotriose to xylohexaose). The results of these enzyme activity assays were evaluated using thin layer chromatography (TLC) (**Fig. 2**

## Claims

1. A polypeptide with at least 85% sequence identity to SEQ ID NO: 1.

2. The polypeptide according to claim 1, wherein the polypeptide has at least 95% sequence identity to SEQ ID NO: 1 or SEQ ID NO: 2;
or wherein the polypeptide comprises the amino acid sequence SEQ ID NO: 1 or SEQ ID NO: 2;
or wherein polypeptide is an endo-β-1,4-xylanase (EC 3.2.1.8).

3. A polynucleotide encoding the polypeptide of claim 1 or 2 with at least 85% sequence identity to SEQ ID NO: 3; or
wherein the polynucleotide has at least 95% sequence identity to SEQ ID NO: 3, SEQ ID NO: 4 or SEQ ID NO:5.

4. A nucleic acid construct or expression vector comprising the polynucleotide of claim 3.

5. A recombinant host cell comprising the polynucleotide of claim 3, or the nucleic acid construct or expression vector of claim 4.

6. A method for obtaining a polypeptide according to claim 1 or 2 comprising:
A) providing a polynucleotide of claim 3,
B) cloning said polynucleotide into a nucleic acid construct or expression vector,
C) transfecting said vector obtained by step B) into recombinant host cells;
D) culturing said recombinant host cells obtained by step C);
E) isolating the polypeptide of claim 1 or 2 from said cultured recombinant host cells.

7. The method according to claim 6, wherein the recombinant host cells are Sf9 cells.

8. Use of the polypeptide of claim 1 or 2 for producing xylose oligosaccharides by transglycosylation reaction; or
for producing xylose oligosaccharides by transglycosylation reaction, wherein the xylose oligosaccharides are xylooligosaccharides (XOS), arabinoxylan oligosaccharides (AXOS), or fragments thereof.

9. A method for producing xylose oligosaccharides comprising:
performing transglycosylation reaction of a glycosyl donor with a glycosyl acceptor by using the polypeptide of claim 1 or 2,
wherein the glycosyl donor is selected from the group consisting of: xylose, xylobiose, xylotriose, xylotetraose, xylopentaose, xylohexaose and
xyloheptaose; and
the glycosyl acceptor is a substrate containing at least 2 xylose units.

10. The method according to claim 9, wherein the substrate containing at least 2 xylose units has the formula (II): 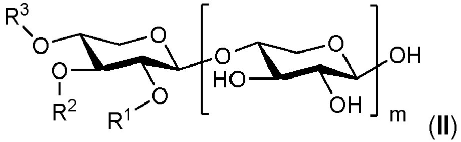 wherein,
**m** is an integer selected from 1 to 6;
**R¹** represents-H, or 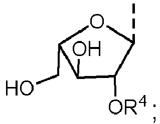
**R²** represents -H, or 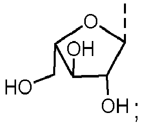
**R³** represents -H, or 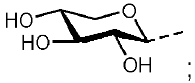
**R⁴** represents -H, or

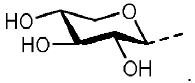

11. The method according to claim 9 or 10, wherein each xylose oligosaccharide has the formula (I): 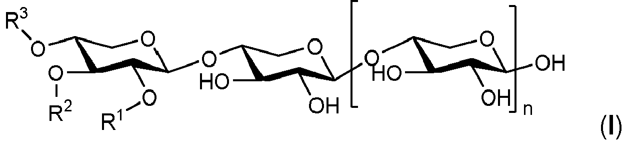 wherein
n is an integer selected from 1 to 18;
**R¹** represents-H, or 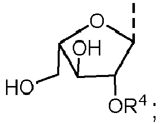
**R²** represents -H, or 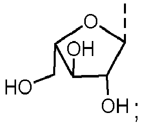
**R³** represents -H, or 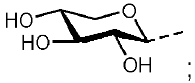
**R⁴** represents -H, or

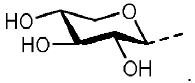

12. A mixture of xylose oligosaccharides obtainable by the method of any one of claims 9 to 11; or
a mixture of xylose oligosaccharides obtainable by the method of any one of claims 9 to 11, wherein the xylose oligosaccharides are selected from xylooligosaccharides (XOS), arabinoxylan oligosaccharides (AXOS), or fragments thereof.

13. The mixture of xylose oligosaccharides according to claim 12, wherein each xylose oligosaccharide has the formula (I): 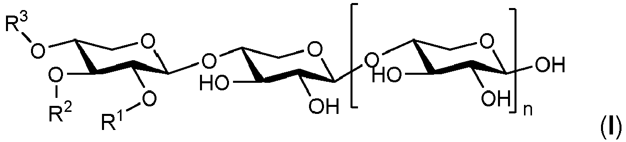 wherein
n is an integer selected from 1 to 18;
**R¹** represents-H, or 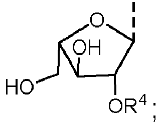
**R²** represents -H, or 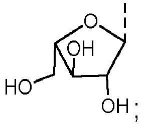
**R³** represents -H, or 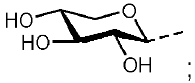
**R⁴** represents -H, or

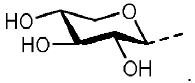

14. The mixture of xylose oligosaccharides according to claim 12 or 13, wherein the mixture comprises or consists of: xylooligosaccharides (XOS) **3** - **20**, and/or arabinoxylan oligosaccharides (AXOS) **23** - **130**;
or wherein the mixture comprises or consists of: xylotriose (**3**), xylotetraose (**4**), xylopentaose (**5**), xylohexaose (**6**), xyloheptaose (**7**), xylooctaose (**8**), xylononaose (**9**), xylodecaose (**10**), xyloundecaose (**11**), xylododecaose (**12**), xylotridecaose (**13**), xylotetradecaose (**14**), and xylopentadecaose (**15**);
or wherein the mixture comprises or consists of: arabinoxylan oligosaccharides (AXOS) **23** - **46**.

15. Use of a mixture of xylose oligosaccharides according to any one of the claims 12 to 14 in chemical analytics and enzymology;
or a mixture of xylose oligosaccharides according to any one of the claims 12 to 14 for use as prebiotics, nutraceuticals, pharmaceutically active ingredients, or food supplements;
or for use in prophylaxis and/or treatment of bacterial infectious disease, cancer, immune disease, type 2 diabetes, or cardiovascular disease.
